# EUROPEAN PATENT SPECIFICATION

(11) **EP 3 322 980 B1**
(45) Date of publication and mention of the grant of the patent: **09.09.2020**
(21) Application number: 16739131.7
(22) Date of filing: 14.07.2016
(51) Int. Cl.: C12Q 1/6883, G01N 33/68, G01N 33/92, A61K 31/137, A61K 31/385, A61K 45/06, A61K 31/397, C12Q 1/48, A61K 31/198, A61K 31/355

(54) **METHODS AND COMPOSITIONS FOR THE DIAGNOSIS AND FOR THE TREATMENT OF ADRENOLEUKODYSTROPHY**
VERFAHREN UND ZUSAMMENSETZUNGEN ZUR DIAGNOSE UND ZUR BEHANDLUNG VON ADRENOLEUKODYSTROPHIE
PROCÉDÉS ET COMPOSITIONS POUR LE DIAGNOSTIC ET LE TRAITEMENT DE L'ADRÉNOLEUCODYSTROPHIE

(30) Priority: 14.07.2015 EP 15382366
(43) Date of publication of application: 23.05.2018
(73) Proprietor: Fundació Institut D'Investigació Biomèdica de Bellvitge (IDIBELL), 08908 Hospitalet de Llobregat-Barcelona (ES); Fundació Institució Catalana de Recerca I Estudis Avançats (ICREA), 08010 Barcelona (ES); Administración General de la Comunidad Autónoma de Euskadi, 01010 Vitoria-Gasteiz, Álava (ES); Universitat de Lleida, 25003 Lérida (ES); Institut de Recerca Biomèdica de Lleida Fundació Dr Pifarré, 25198 Lérida (ES)
(72) Inventor: PUJOL ONOFRE, Aurora, E-08860 Sitges-Barcelona (ES); PORTERO OTÍN, Manuel, E-25003 Lérida (ES); PAMPLONA GRAS, Reinaldo, E-25003 Lérida (ES); LÓPEZ DE MUNAIN ARREGI, Adolfo José, E-20014 San Sebastián - Guipúzcoa (ES); JOVÉ FONT, Mariona, E-25198 Lérida (ES); FOURCADE, Stephane, E-08003 Barcelona (ES)
(74) Representative: ABG Intellectual Property Law, S.L.
(86) International application number: PCT/EP2016/066825
(87) International publication number: WO 2017/009437

(56) References cited:
- EP-A1- 2 527 344
- WO-A1-2012/112933
- WO-A1-2014/110154
- WO-A1-2014/168986
- US-A1- 2010 260 755
- DI BIASE A ET AL: "Th 1 cytokine production by peripheral blood mononuclear cells in X-linked adrenoleukodystrophy.", JOURNAL OF THE NEUROLOGICAL SCIENCES 1 JAN 2001, vol. 182, no. 2, 1 January 2001 (2001-01-01), pages 161-165, XP002752733, ISSN: 0022-510X

## Description

### FIELD OF THE INVENTION

The present invention belongs to the field of diagnostic and therapeutics and, more in particular, to the diagnosis and treatment of adrenoleukodystrophy.

### BACKGROUND OF THE INVENTION

With an incidence of 1 in 17,000 in newborns, X-linked adrenoleukodystrophy (X-ALD, OMIM number 300100) is the most common monogenic leukodystrophy and peroxisomal disorder. X-ALD is characterized by central inflammatory demyelination in the brain and/or slowly progressing spastic paraparesis resulting in axonal degeneration in the spinal cord. X-ALD is caused by mutations in the *ABCD1* gene (Xq28), which encodes the ATP-binding cassette transporter, an integral peroxisomal membrane protein involved in the import of very long-chain fatty acids (C≥22:0) and very long-chain fatty acids -CoA esters into the peroxisome for degradation. The defective function of the ABCD1 transporter leads to very long-chain fatty acids accumulation and impaired β-oxidation of very long-chain fatty acids in organs and tissues, particularly hexacosanoic acid (C26:0), the pathognomonic disease marker.

Three major disease variants have been described. One is a late-onset form affecting adults, which is known as adrenomyeloneuropathy (AMN). Patients present with peripheral neuropathy and distal axonopathy involving corticospinal tracts of spinal cord,-but without signs of overt inflammatory demyelination- and spastic paraparesis as main symptoms. This form can evolve ultimately into a lethal form, with cerebral inflammatory demyelination in adults, cAMN. The childhood cerebral form cALD has a similar outcome. For cALD patients, the only treatment to date is allogeneic bone marrow transplantation, which is associated with a high morbidity and mortality and is available only to nearly asymptomatic X-ALD children. Very recently, a gene therapy approach correcting CD34+ cells with the *ABCD1* cDNA, using a lentiviral vector, has proved to be successful and, being less invasive, is a good alternative to transplantation.

In contrast, for AMN patients, there is no satisfactory treatment to date. Studies on pathomechanisms underlying disease posit that the excess of C26:0 disturbs mitochondrial oxidative phosphorylation (OXPHOS) function and elicits mitochondrial ROS. This interferes with mitochondrial biogenesis and mitochondrial calcium signaling, which underline a cross-talk between mitochondria and peroxisomes and pinpoint a secondary mitochondrial involvement as culprit in this disease. The identification of targets in these pathways, such as PGC-1α, Sirt1, or mTOR, which can be targeted with drugs, has led to successful preclinical tests in the *Abcd1-* mouse model (a model for AMN), which warrant clinical trials. Although the entire clinical spectrum of X-ALD is initiated by mutations in a single gene, the *ABCD1,* the pathomechanisms for demyelination, the inflammatory process, the axonal degeneration and the adrenal insufficiency clearly differ. Therefore, additional pathogenic factors critically shaping the clinical manifestation of X-ALD /XAMN ought to exist. Some of the molecular pathology present in nervous tissue can be reproduced in fibroblasts or other cells by incubation with excess of C26:0, including oxidative stress or dysfunction of proteasomal and mitochondrial compartments. Despite this finding, it is not clear how a single defect in peroxisomal fatty acid metabolism could elicit such a varied neurologic phenotype.

Therefore, there is a need for new biomarkers of the disease allowing not only diagnosing the disease but also monitoring the progression of the disease and the effect of a therapy.

### SUMMARY OF THE INVENTION

The inventors have surprisingly found that patients suffering from X-ALD show an increase in the sphingosine kinase (SPHK) route which results in increased levels of the SPHK2 enzyme and in the sphingosine-1-phosphate receptor 1 as well as in increased levels of the SPHK product sphingosine-1-phosphate (SIP) (Example 1). Moreover, combining an untargeted metabolome assay of X-ALD patients with a functional genomics analysis of spinal cords of Abcd1⁻ mouse, the inventors have identified a series of biomarkers with altered levels in X-ALD patients (Example 2). This allows the use of the levels of these molecules for the diagnosis of X-ALD as well as for monitoring progression of the disease or for determining whether an X-ALD patient responds to a therapy.

Thus, in a first aspect, the invention relates to a method for the diagnosis of an adrenoleukodystrophy in a subject comprising determining in a sample from said subject a value selected from the group consisting of
- the levels of sphingosine-1-phosphate,
- the expression levels of sphingosine kinase 2,
- the expression levels of sphingosine-1-phosphate receptor,
   wherein
   increased levels of sphingosine-1-phosphate,
   increased levels of sphingosine kinase 2,
   increased levels of sphingosine-1-phosphate receptor,
   with respect to a reference value are indicative that the subject suffers from an adrenoleukodystrophy, and
   wherein if said value is the expression levels of tumour necrosis factor A, then the adrenoleukodystrophy occurs without inflammatory demyelination.

In a second aspect, the invention relates to a method for monitoring the progression of an adrenoleukodystrophy in a subject suffering from adrenoleukodystrophy comprising determining in a sample from said subject a value selected from the group consisting of
- the levels of sphingosine-1-phosphate,
- the expression levels of sphingosine kinase 2,
- the expression levels sphingosine-1-phosphate receptor,
wherein
increased levels of sphingosine-1-phosphate,
increased levels of sphingosine kinase 2,
increased levels of sphingosine-1-phosphate receptor,
with respect to a value determined in a sample from the same subject at an earlier time point is indicative of a worsening of the adrenoleukodystrophy or wherein
decreased levels of sphingosine-1-phosphate,
decreased levels of sphingosine kinase 2,
decreased levels of sphingosine-1-phosphate receptor,
with respect to a value determined in a sample from the same subject at an earlier time point t is indicative of an amelioration of the adrenoleukodystrophy.

In a third aspect, the invention relates to a method for monitoring the effect of an adrenoleukodystrophy therapy in a subject suffering from adrenoleukodystrophy comprising determining in a sample from said subject a value selected from the group consisting of
- the levels of sphingosine-1-phosphate,
- the expression levels of sphingosine kinase 2,
- the expression levels of sphingosine-1-phosphate receptor,
wherein
increased levels of sphingosine-1-phosphate,
increased levels of sphingosine kinase 2,
increased levels of sphingosine-1-phosphate receptor,
with respect to the value determined prior to the administration of the therapy is indicative that the therapy is not effective
or wherein
decreased levels of sphingosine-1-phosphate,
decreased levels of sphingosine kinase 2,
decreased levels of sphingosine-1-phosphate receptor,
with respect to a value determined prior to the administration of the therapy is indicative that the therapy is effective.

In addition, the inventors have also found that treatment of X-ALD patients with antioxidants results in a decrease in the levels of S1P, which opens the door for the treatment of X-ALD using molecules which produce a reduction in S1P levels, such as inhibitors of S1PR1 like, for instance, fingolimod.

Thus, in a fourth aspect the invention relates to an inhibitor of sphingosine-1-phosphate receptor 1 for use in the treatment and/or prevention of adrenoleukodystrophy.

In a fifth aspect, the invention relates to a pharmaceutical composition comprising an inhibitor of sphingosine-1-phosphate receptor 1 and one or more drugs selected from the group consisting of an antioxidant selected from alpha-lipoic acid, vitamin E, and N-acetylcysteine, an antioxidant targeted to mitochondria, a histone deacetylase inhibitor, an inhibitor of mitochondria transition pore opening, an anti-inflammatory drug, a PPAR agonist, a RXR agonist, a sirtuin 1 agonist, an hypolipidemic drug, , a fatty acid composition able to decrease circulating levels of hexacosanoic acid (C26:0) and an autophagy activator.

In a sixth aspect, the invention relates to the pharmaceutical composition of the fifth aspect for use in the treatment and/or prevention of an adrenoleukodystrophy.

### DESCRIPTION OF THE FIGURES

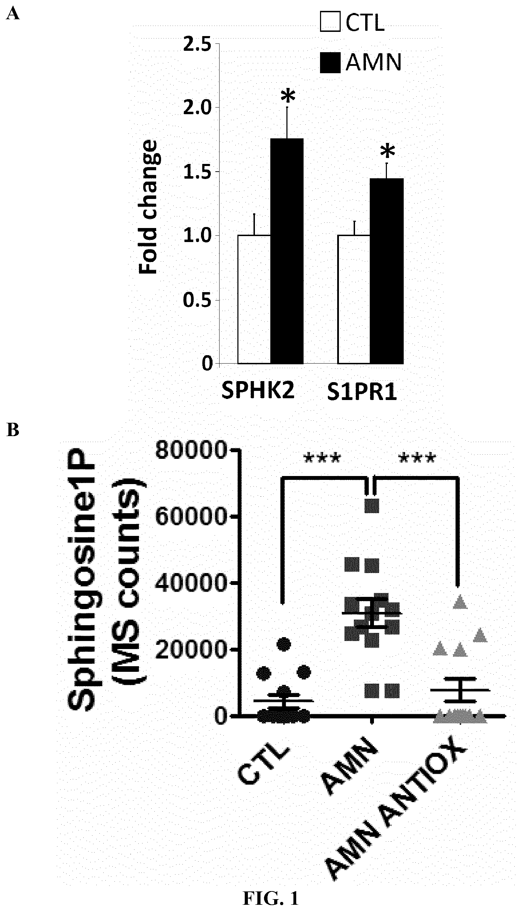

**Figure 1****. A.** Sphingosine-2-phosphase kinase and Sphingosine-1-phosphate receptor 1 levels are increased in peripheral mononuclear cells from AMN patients compared to healthy controls. Q-PCR results. **B.** Sphingosine-1-phosphate levels in peripheral mononuclear cells from AMN patients before and after antioxidant treatment, compared to healthy controls. Levels were analysed using a HPLC 1290 series coupled to an ESI-Q-TOF. Metabolomic results. Significant differences were determined by ANOVA followed by Tukey HSD *post hoc* (**P*<0.05, ***P*<0.01, ****P*<0.001).

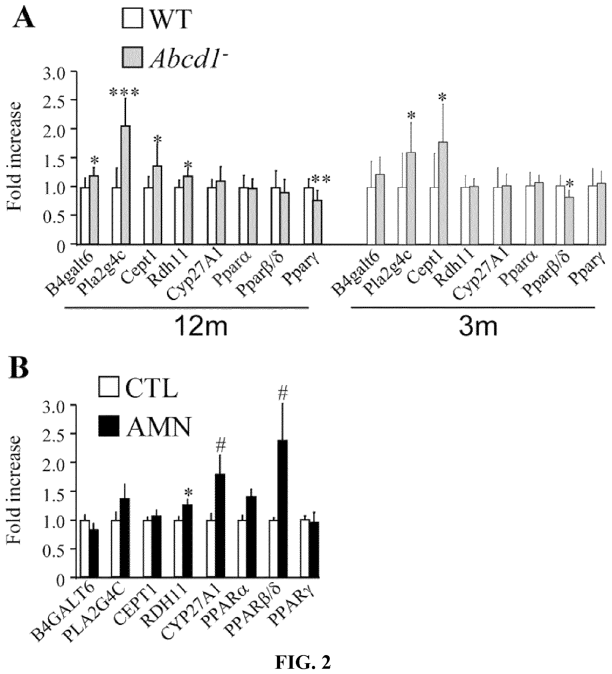

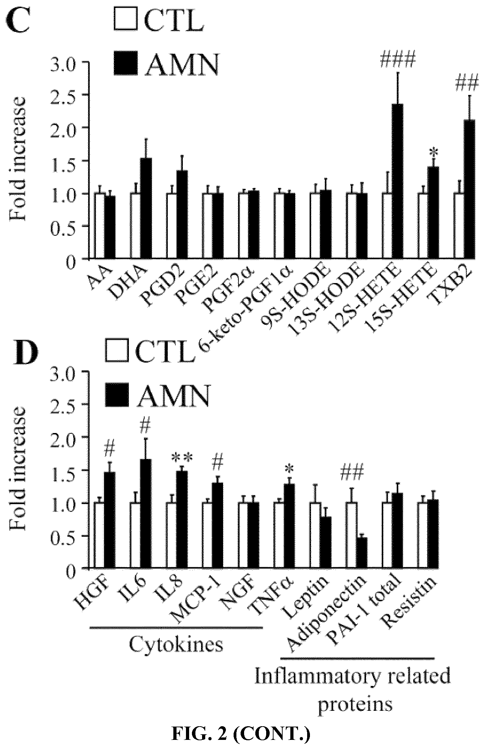

**Figure 2****. Eicosanoids, oxidized polyunsaturated fatty acids and adipokine levels in body fluids from AMN patients** (n=13 AMN and 13 healthy age and sex matched individuals). **(A)** *B4galt6, Pla2g4c, Cept1, Rdh11, Cyp27a1, Ppar*α*, Ppar*β/δ, *Ppar*γ and *Gpx4* gene expression in *Abcd1-* spinal cords at 3.5 and 12 months of age. Gene expression was normalized to the reference control gene mouse *Rpl0.* **(B)** *B4GALT6, PLA2G4C, CEPT1, RDH11, CYP27A1, PPAR***α***, PPAR***β/δ***, PPAR***γ** and *GPX4* gene expression in PBMC from AMN patients and healthy controls. Gene expression was normalized to the reference control gene human *RPL0.* **(C)** Relative levels of the inflammation-associated lipids arachidonic acid (AA), docosahexaenoic acid (DHA), prostaglandins D2, E2 and F2α (PGD2, PGE2, PGF2α), 6-keto-PGF1α, (±) 9-hydroxy-10E, 12Z octadecadienoic acid (9S-HODE), (±) 13(S)-hydroxy-9Z, 11E octadecadienoic acid (13S-HODE), (±)12-, and 15-hydroxy-5Z, 8Z, 11Z, 13Eeicosatetraenoic acid (12S-HETE and 15S-HETE) and thromboxane B2 (TXB2). **(C)** The levels of HGF (hepatocyte growth factor), IL6, IL8, MCP-1 (monocyte chemoattractant protein-1 or CCL2), NGF (nerve growth factor), TNFα, leptin, adiponectin, total PAI-1 (plasminogen activator inhibitor-1) and resistin were quantified in serum from AMN patients and controls by using Milliplex technology. The values are means ± SEM. Significant differences have been determined by one-tail Student's t test (**P*<0.05 and ***P*<0.01) or Wilcoxon rank sum test (^{#}*P*<0.05, ^{##}*P*<0.01 and ^{###}*P*<0.001) according to Shapiro-Wilk normality test.

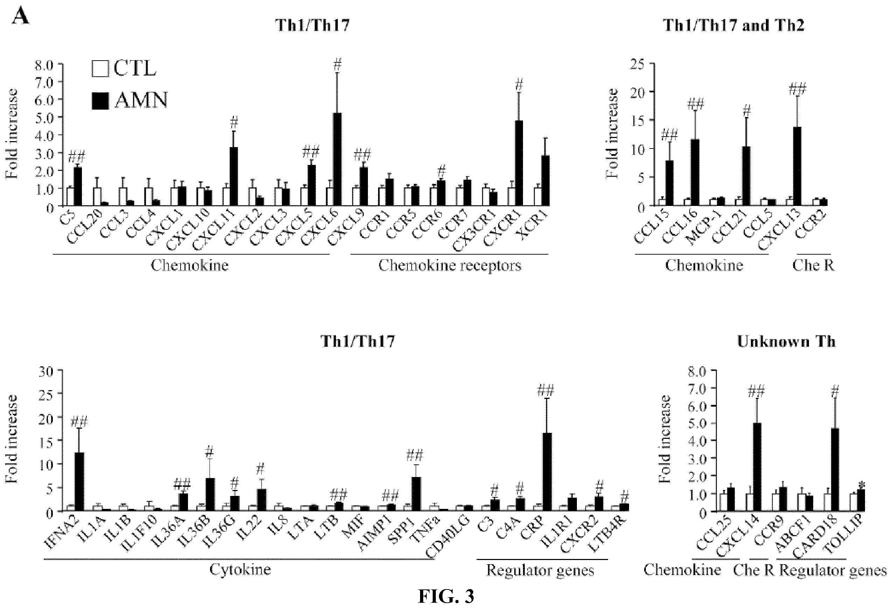

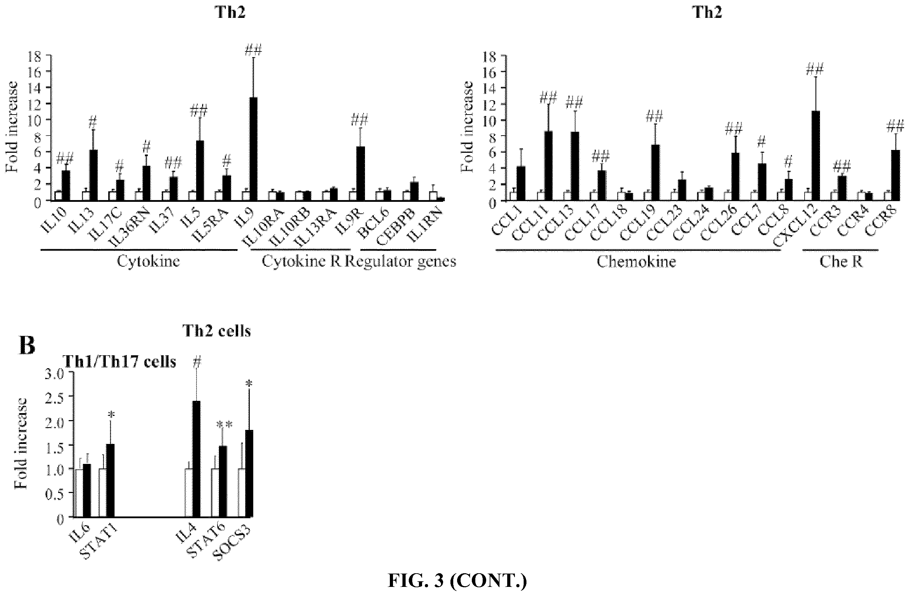

**Figure 3****. Inflammatory cytokines, chemokines and receptors related signaling in PBMC from AMN patients** (n=13 AMN and 13 healthy age and sex matched individuals). (A) Gene expression of 84 genes of the Inflammatory Cytokines and Receptors Signaling Pathway RT2 Profiler Q-PCR Array (Qiagen). Gene expression was normalized to internal controls. **(B)** IL4, IL6, STAT6, SOCS3, and STAT1 gene expression in PBMC from AMN patients and healthy controls. Gene expression was normalized to the reference control human RPL0. Genes have been classified according to their roles in Th2 and Th1/Th17 polarization. The values represent mean ± SEM. Significant differences have been determined by one-tail Student's *t*-test (**P*<0.05 and ***P*<0.01) or Wilcoxon rank sum test (#*P*<0.05 and ##P<0.01) according to Shapiro-Wilk normality test.

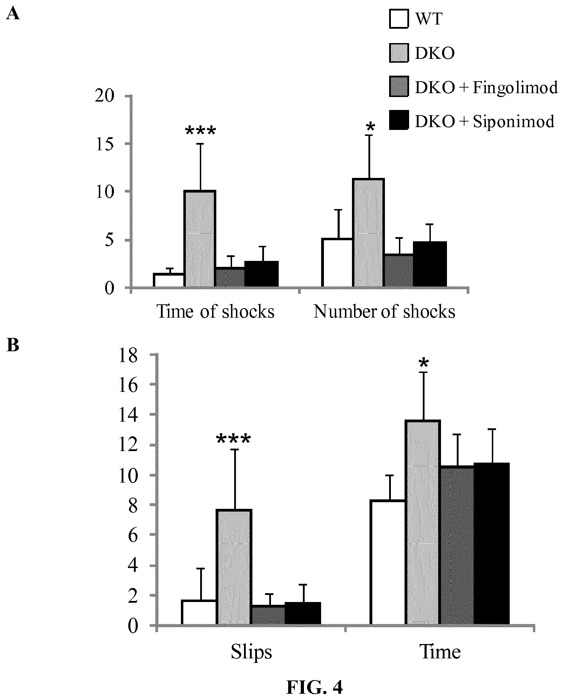

**Figure 4****. Fingolimod and siponimod prevent locomotor disability in *Abcd1⁻*/*Abcd2*^{*-*/*-*} mice**
   **(A-B)** Treadmill **(A)** and bar cross **(B)** tests were conducted on WT, *Abcd1⁻*/*Abcd2*^{*-*/*-*} (DKO), fingolimod-treated *Abcd1⁻*/*Abcd2^{-l-}* (DKO + Fingolimod) and siponimod-treated *Abcd1⁻*/*Abcd2*^{*-*/*-*} (DKO + Siponimod) of 16 months of age, treated for 4 months starting at 12 months of age. **(A)** The mean (Mean) and the best performance (Maximum) score of each animal was used for statistical analysis. The latency to falling from the belt (time of shocks) and the number of shocks received were computed after 5 min. **(B)** The time spent to cross the bar and the number of slips were quantified. Values are expressed as the mean ± SD (n=16 per condition in **A-C;** **P*<0.05, ***P*<0.01 and ****P*<0.001, one-way ANOVA followed by Tukey's *HSD post hoc* test).

### DESCRIPTION OF THE INVENTION

### Diagnostic method of the invention

In a first aspect, the invention relates to a method for the diagnosis of an adrenoleukodystrophy in a subject, hereinafter diagnostic method of the invention or first method of the invention, comprising determining in a sample from said subject a value selected from the group consisting of
- the levels of sphingosine-1-phosphate,
- the expression levels of sphingosine kinase 2,
- the expression levels sphingosine-1-phosphate receptor,
wherein
increased levels of sphingosine-1-phosphate,
increased levels of sphingosine kinase 2,
increased levels of sphingosine-1-phosphate receptor,
with respect to a reference value are indicative that the subject suffers from an adrenoleukodystrophy and
wherein if said value is the expression level of tumour necrosis factor A, then the adrenoleukodystrophy occurs without inflammatory demyelination.

The term "diagnosis", as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. In particular, the term "diagnosis of an adrenoleukodystrophy" relates to the capacity to identify or detect the presence of adrenoleukodystrophy in a subject. This diagnosis, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical tools; illustrative, non-limiting examples of said statistical tools include determining confidence intervals, determining the p-value, the Student's t-test or Fisher's discriminant functions, etc. (see, for example, Dowdy and Wearden, Statistics for Research, John Wiley & Sons, New York 1983). The confidence intervals are preferably at least 90%, at least 95%, at least 97%, at least 98% or at least 99%. The p-value is preferably less than 0.1, less than 0.05, less than 0.01, less than 0.005 or less than 0.0001. The teachings of the present invention preferably allow correctly diagnosing in at least 60%, in at least 70%, in at least 80%, or in at least 90% of the subjects of a determined group or population analyzed.

The terms "adrenoleukodystrophy" or "X-linked adrenoleukodystrophy" or "ALD" or "X-ALD", as used herein, refer to a monogenic leukodystrophy (group of disorders that are characterized by an abnormal formation, turnover, or destruction of myelin) that leads to progressive damage to the brain, adrenal gland, peripheral nervous system, and eventually death. In a particular embodiment the adrenoleukodystrophy is selected from the group consisting of:
- adult adrenomyeloneuropathy (AMN): late onset form characterized by peripheral neuropathy and distal axonopathy in spinal cords but without signs of inflammatory demyelination,
- cerebral adrenomyeloneuropathy with brain inflammatory demyelination (cAMN) and
- a childhood cerebral variant (cALD), characterized by severe cerebral inflammatory demyelination.

In a more preferred embodiment, the adrenoleukodystrophy is adrenomyeloneuropahy (AMN).

When the value determined according to the method of diagnosis of the disclosure is the expression level of TNFA, that is, when the biomarker is TNFA, the ALD occurs without inflammatory demyelination. When the value determined according to the method of diagnosis of the invention is not the expression level of TNFA, that is, when the biomarker is not TNFA, the ALD can occur with or without inflammatory demyelination. In a particular embodiment, the ALD occurs without inflammatory demyelination.

The term "inflammatory demyelination", as used herein, refers to a pathological condition characterized by a damage or destruction of neural tissue (such as without limitation cells of the central nervous system, including e.g., neuronal or glial cells (e.g. oligodendrocytes), or their specific fragments, such as neurites, axons, or myelin) resulting from induction and infiltration of peripheral immune cells into the brain parenchyma. In particular, inflammatory demyelination may be provoked by the activation of microglial cells releasing proinflammatory cytokines and/or by the activation of T and/or B cells and/or monocytes/macrophages.

The term "subject" relates to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age, sex or race.

The term "sample", as used herein, refers to biological material isolated from a subject. The biological sample contains any biological material suitable for detecting RNA, protein or lipid levels. In a particular embodiment, the sample comprises genetic material, e.g., DNA, genomic DNA (gDNA), complementary DNA (cDNA), RNA, heterogeneous nuclear RNA (hnRNA), mRNA, etc., from the subject under study. The sample can be isolated from any suitable tissue or biological fluid such as, for example blood, saliva, plasma, serum, urine, cerebrospinal liquid (CSF), feces, a surgical specimen, a specimen obtained from a biopsy, and a tissue sample embedded in paraffin. In a particular embodiment, the sample from the subject according to the methods of the present invention is selected from the group consisting on serum, plasma and a sample containing peripheral blood mononuclear cells or "PBMC". In a more particular embodiment, the sample is a sample containing PBMC. In an even more particular embodiment, the sample containing PBMC is blood. In a particular embodiment, when the marker is adiponectin or one of the markers defined in Table 1, Table 2, Table 6 or Table 7, the sample is serum or plasma. In another particular embodiment, when the sample is sphingosine-1-phosphate, sphingosine-2-phopshate kinase, sphingosine-1-phosphate receptor or one of the markers defined in Table 3, Table 4, Table 5 or Table 8, the sample is a sample comprising PBMC.

The term "marker" or "biomarker", as used herein, refers to a biomolecule, such as a protein, a nucleic acid, a lipid, a carbohydrate or a metabolite, the occurrence or amount of which is characteristic for a specific situation, for example, an ALD.

The markers useful for the diagnostic method of the invention are:

### • Sphingosine-1-phosphate

The term "sphingosine-1-phosphate" or "SIP" or "lysosphingolipid", as used herein, refers to a signalling sphingolipid of formula:

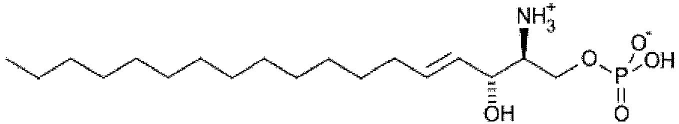

### • Sphingosine kinase 2

The term "sphingosine kinase 2" or *"SPHK2",* as used herein, refers to a gene encoding the nuclear isoform of an enzyme that catalyzes the phospholyration of sphingosine to form sphingosine-1-phosphate. In humans, the gene corresponds to GenBank Gene ID 56848 (release of 4 May 2015) and the protein encoded by said gene is defined in the UniProtKB/Swiss-Prot database with accession number Q9NRA0 (release of 29 April 2015).

### • Sphingosine-1-phosphate receptor 1

The term "sphingosine-1-phosphate receptor 1" or *"SIPR1",* as used herein, refers to a gene encoding a G protein-coupled receptor for sphingosine-1-phosphate. In humans, the gene corresponds to GenBank Gene ID 8879 (release of 4 May 2015) and the protein encoded by said gene is defined in the UniProtKB/Swiss-Prot database with accession number P21453 (release of 29 April 2015).

### • Adiponectin

The term "adiponectin" or *"ADIPOQ",* as used herein, refers to gene encoding an adipokine involved in the control of fat metabolism and insulin sensitivity. In humans, the gene corresponds to GenBank Gene ID 9370 (release of 17 May 2015) and the protein encoded by said gene is defined in the UniProtKB/Swiss-Prot database with accession number Q 15848 (release of 29 April 2015).

### • Neopterin

The term "neopterin", as used herein, refers to a pteridine of formula:

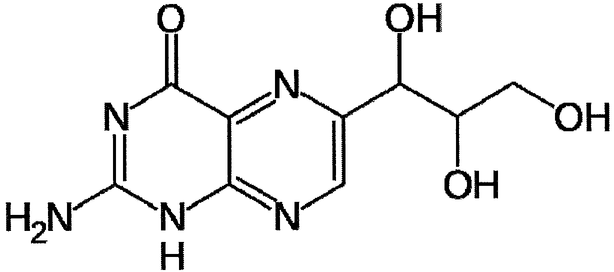

### • Markers defined in Table 1

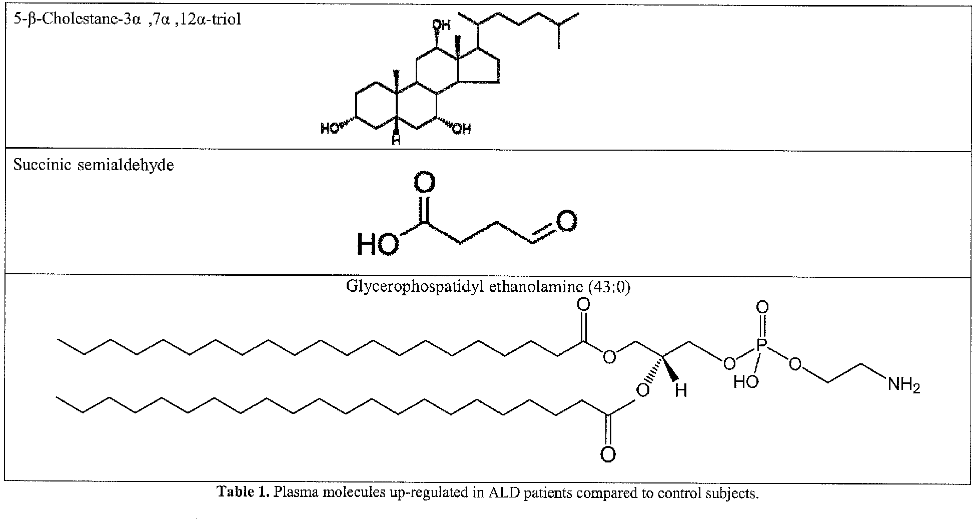

### • Markers defined in Table 2

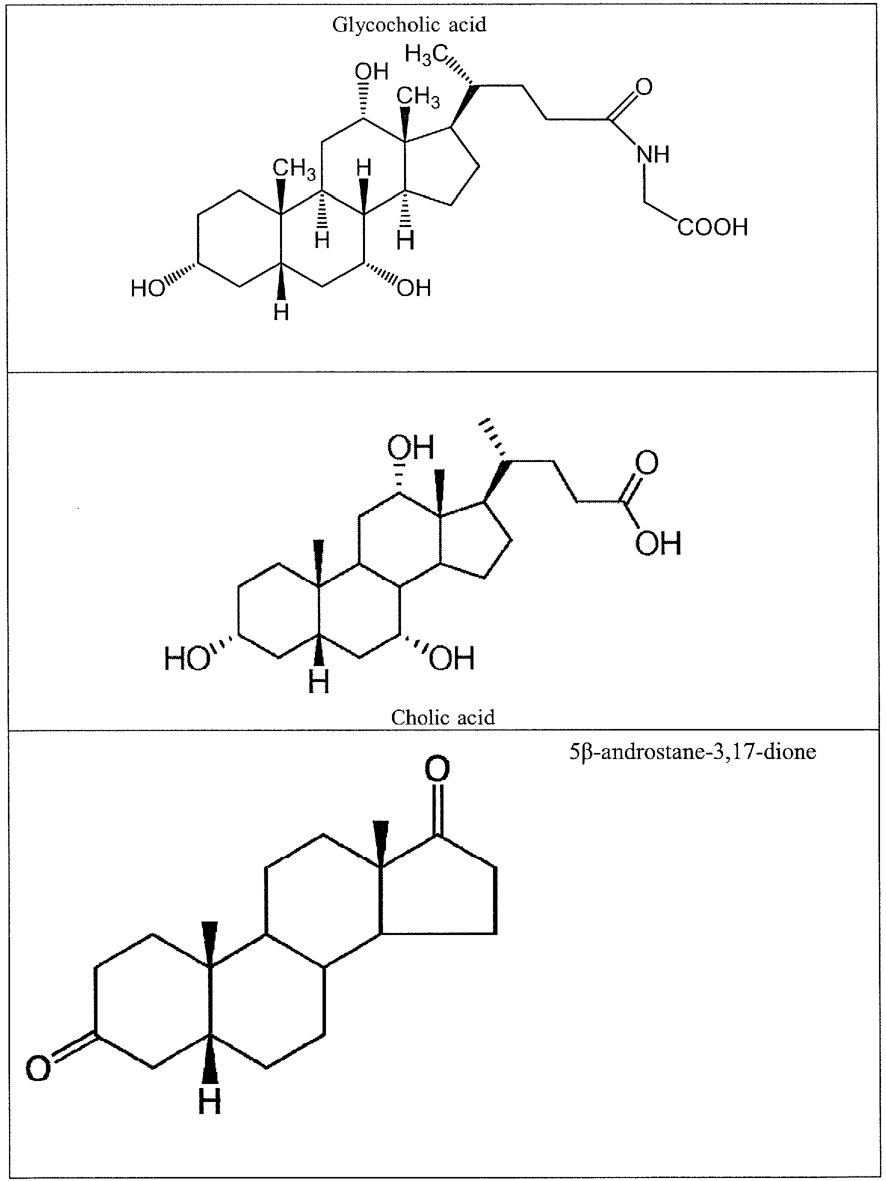

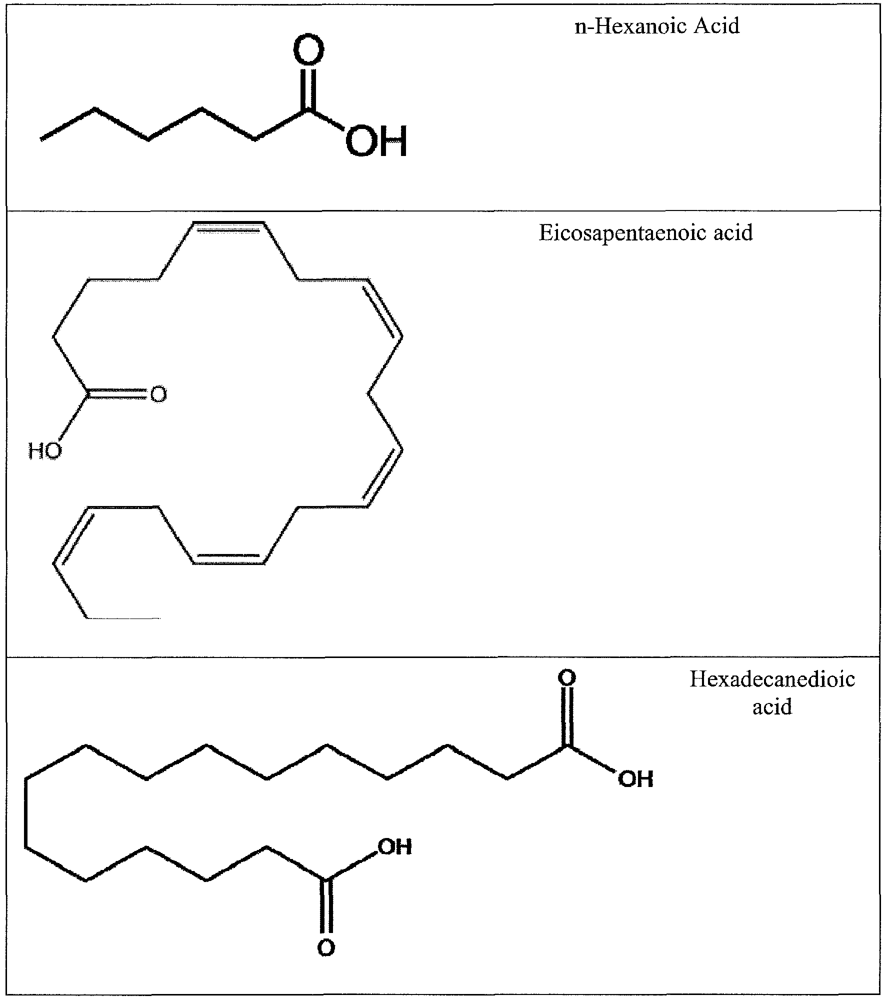

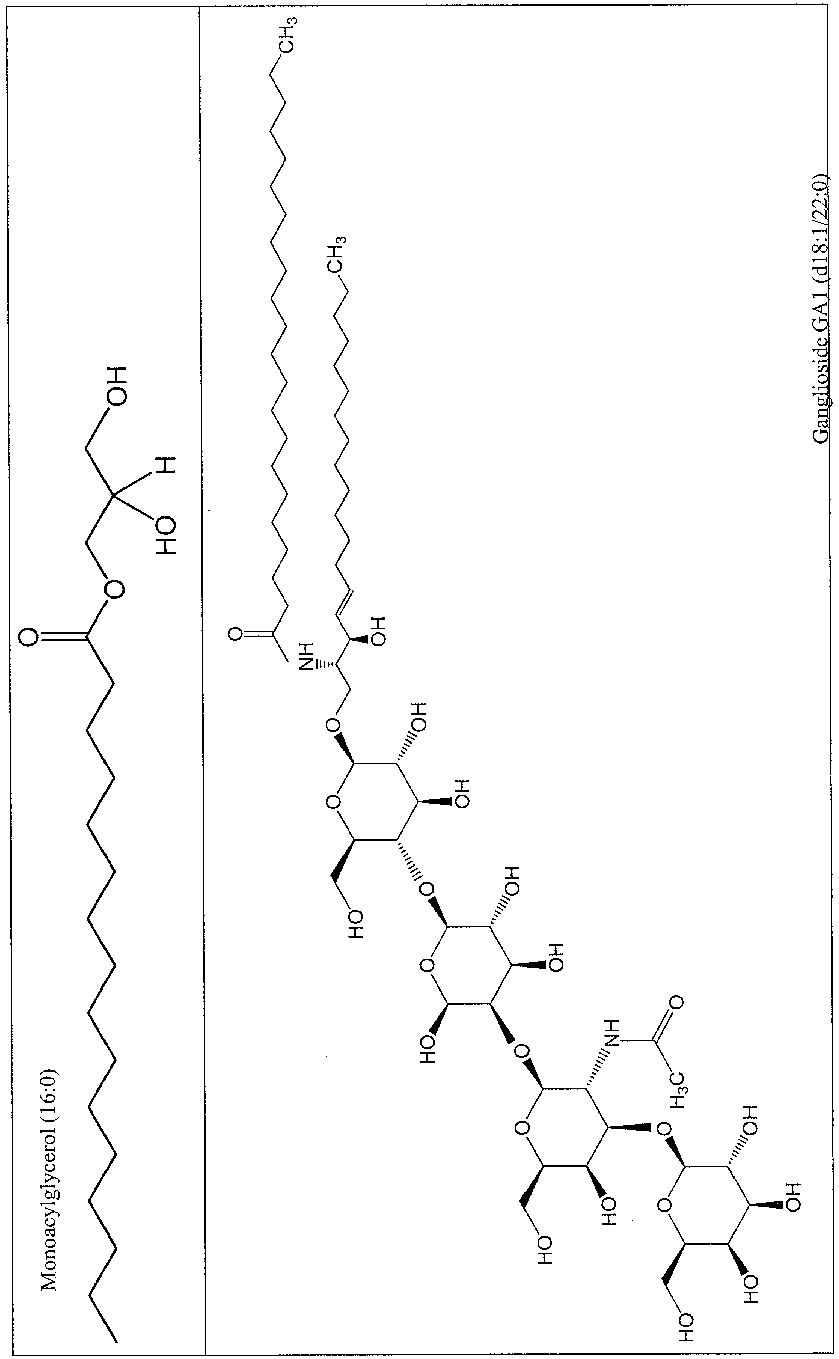

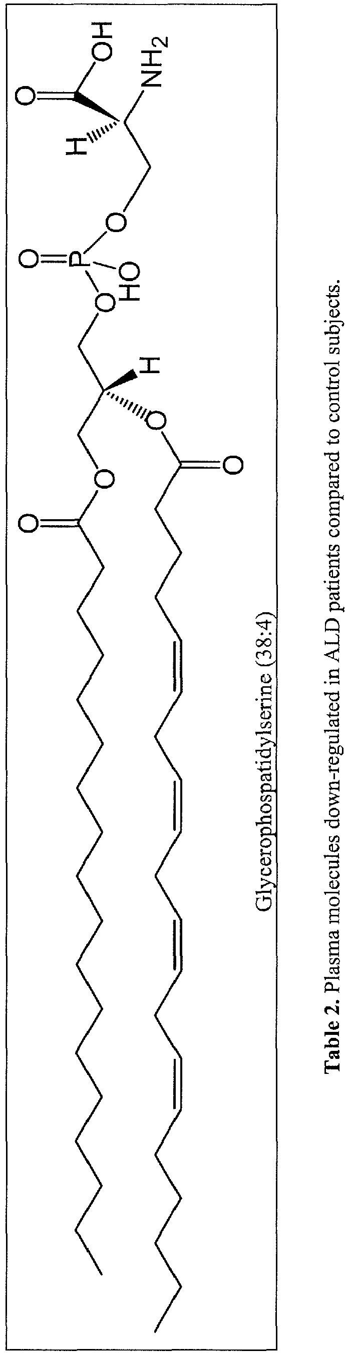

### • Markers defined in Table 3

**Table 3. Molecules up-regulated in PBMC in ALD patients compared to control subjects.**

| **Marker** | **Structure/molecular composition** |
|---|---|
| 1-methylhistidine | 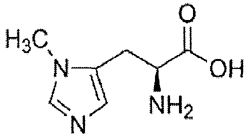 |
| 1-monopalmitin | 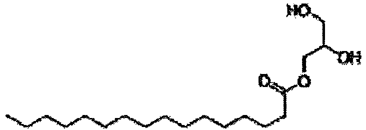 |
| 3-amino-1-tyrosine | 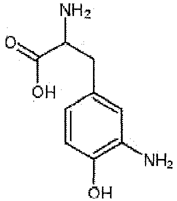 |
| 3-mercaptopyruvate | 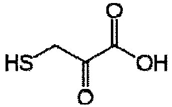 |
| Cytidinediphosphate-ethanolamine | 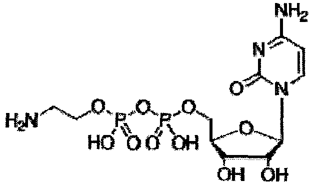 |
| Dehydroascorbic acid | 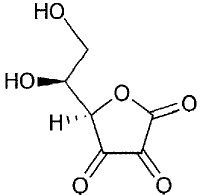 |
| Elaidic acid | 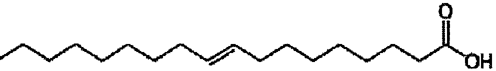 |
| Erythritol | 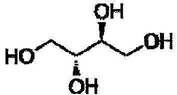 |
| Arachidonic acid | 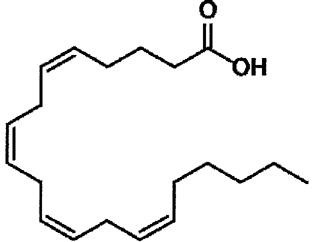 |
| Galactonic acid | 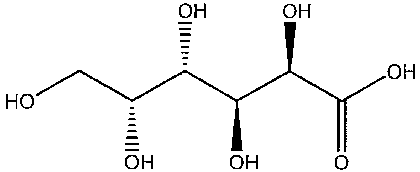 |
| Glutathione | 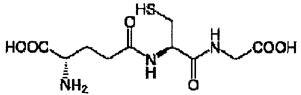 |
| Histamine | 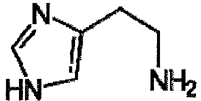 |
| Hypoxanthine | 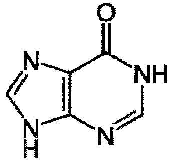 |
| Caprolactone | 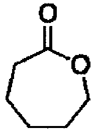 |
| Lactamide | 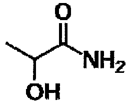 |
| Methyl linolenate | 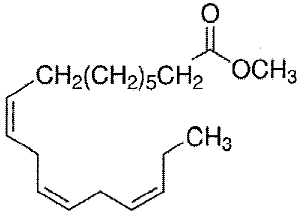 |
| Methyl oleate | 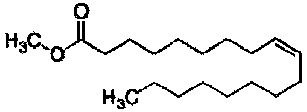 |
| 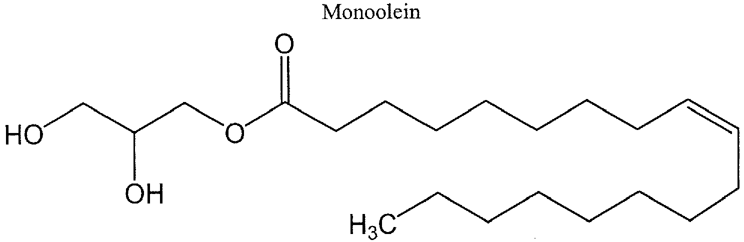 | |
| 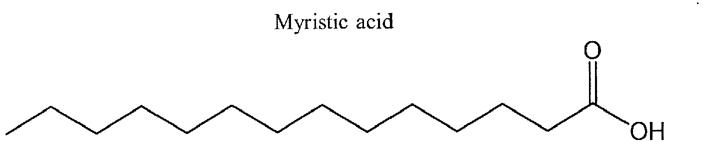 | |
| 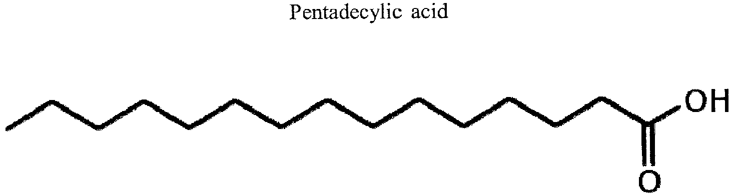 | |
| 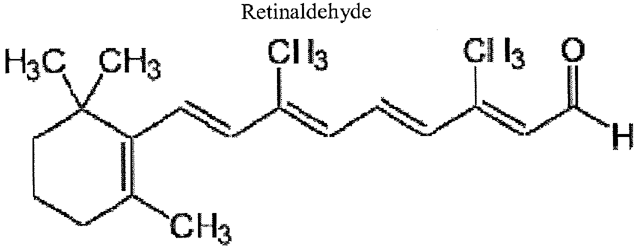 | |
| 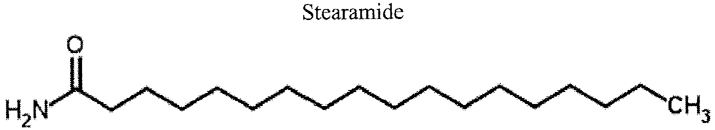 | |
| 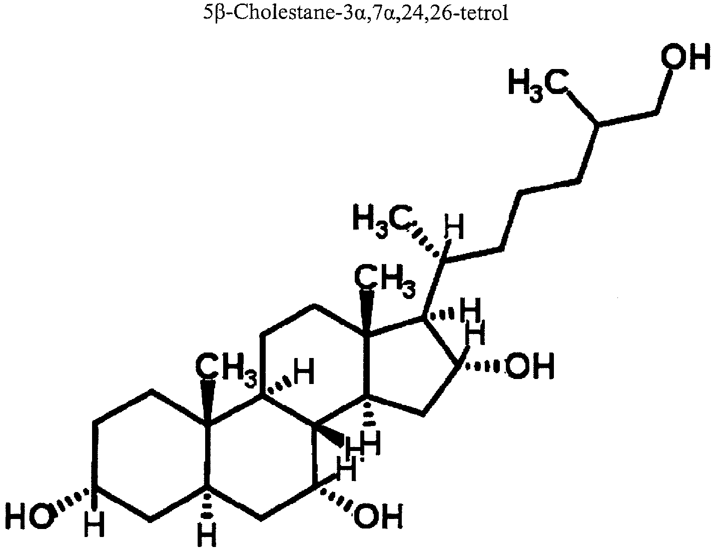 | |
| 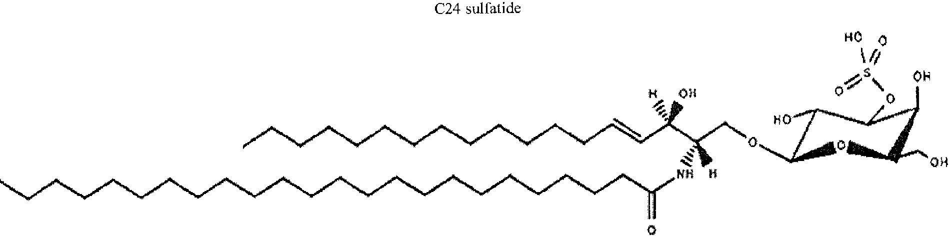 | |
| Cholesteryl ester 19:0 C₄₆H₈₂O₂ | |
| Glycerophospatidylethanolamine (26:0) C₃₁H₆₂NO₈P | |
| 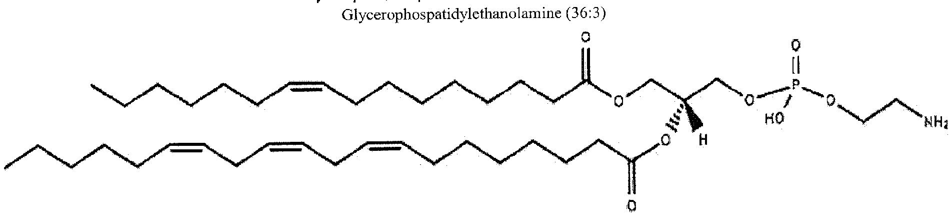 | |
| 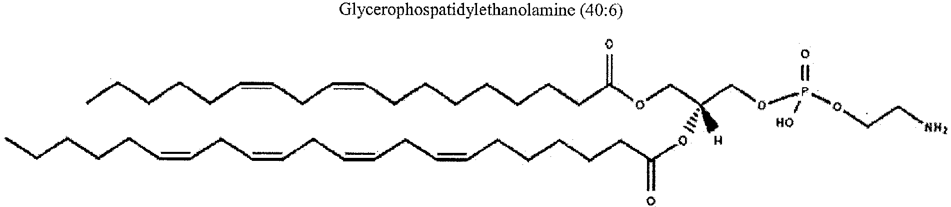 | |
| 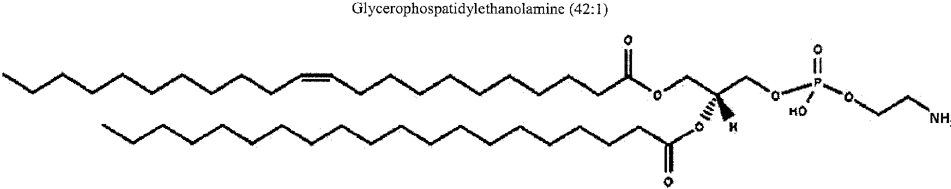 | |
| Glycero phospatidylethanolamine (43:6) C₄₉H₈₄NO₈P | |
| 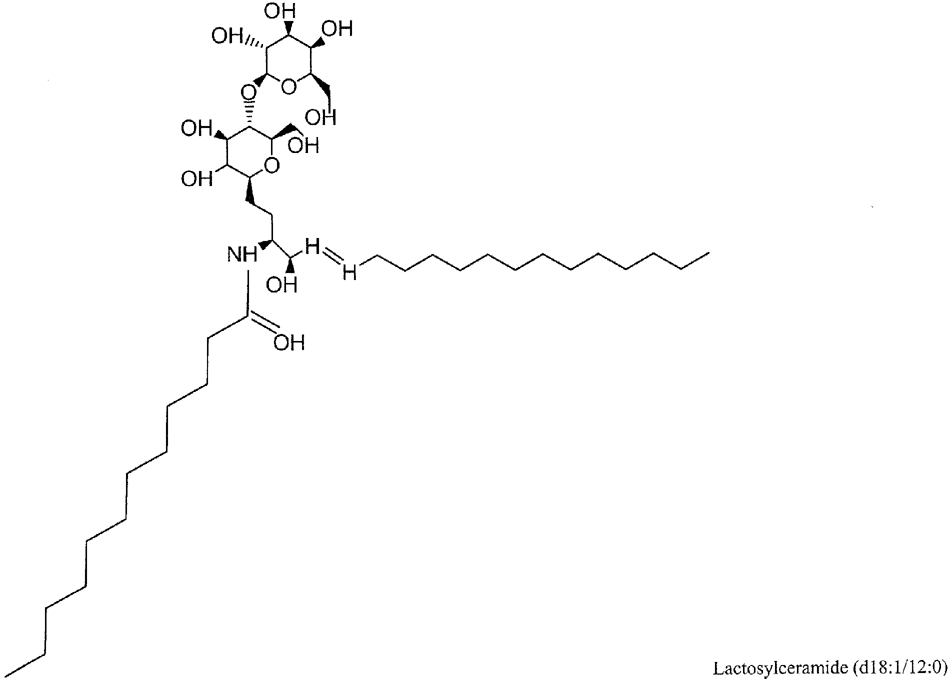 | |
| 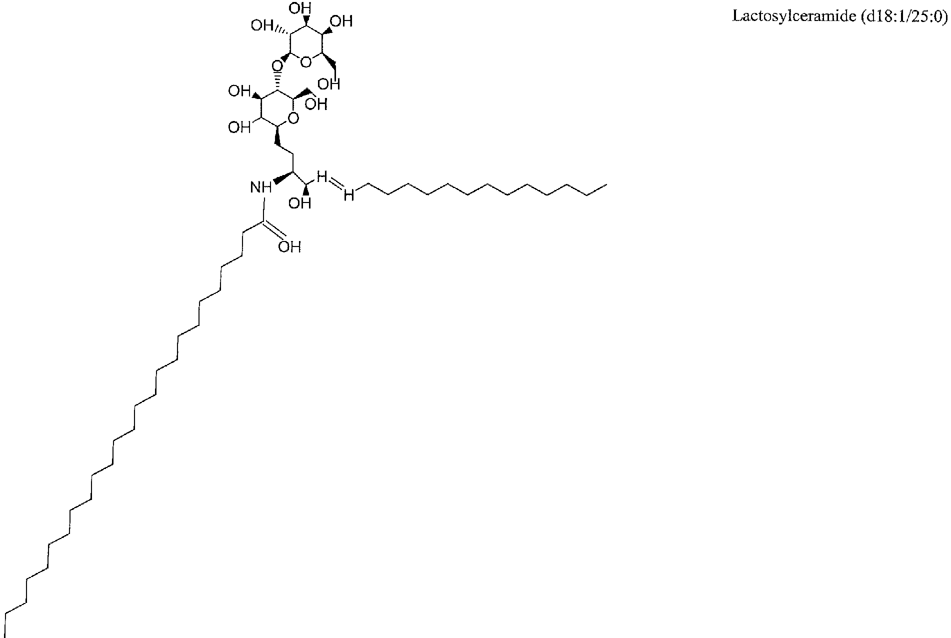 | |
| Triacylglycerol (40:0) C₄₃H₈₃O₆ | |
| 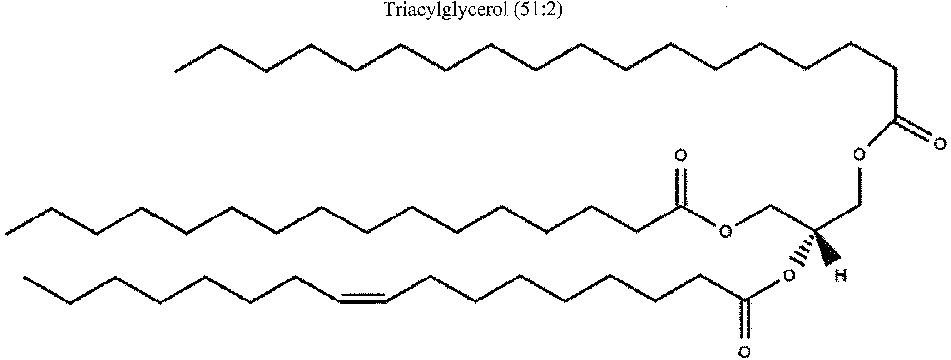 | |
| 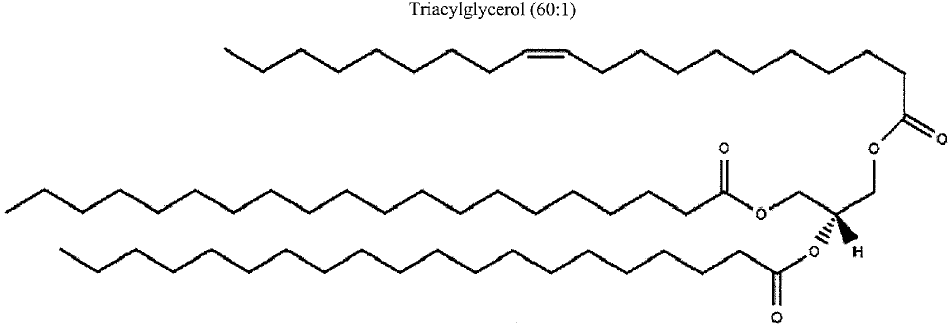 | |
| 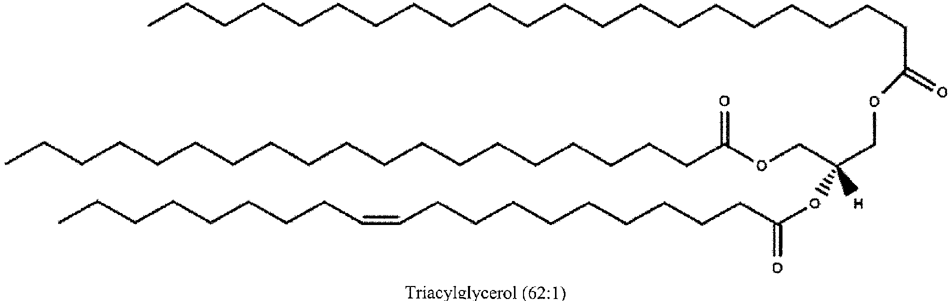 | |
| 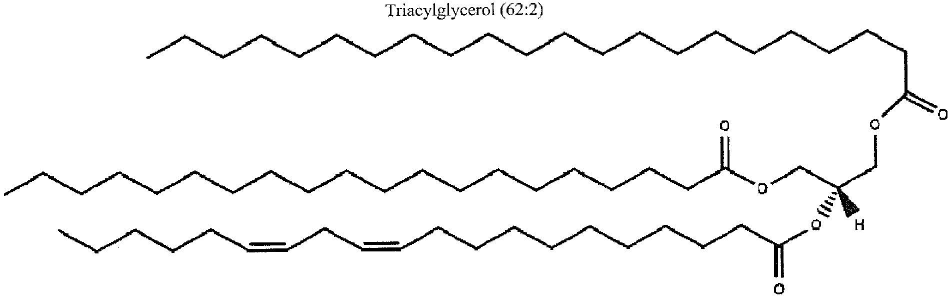 | |
| 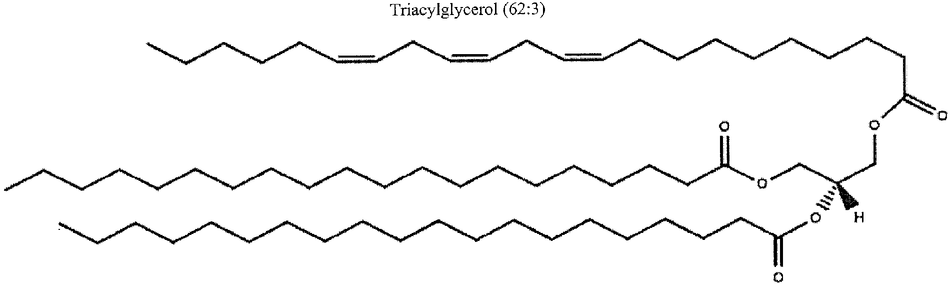 | |
| 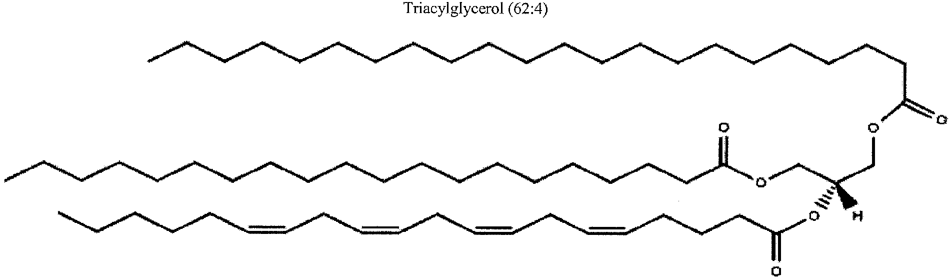 | |
| 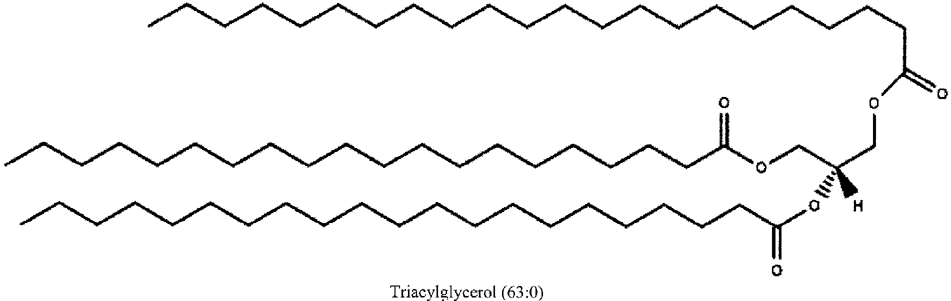 | |
| 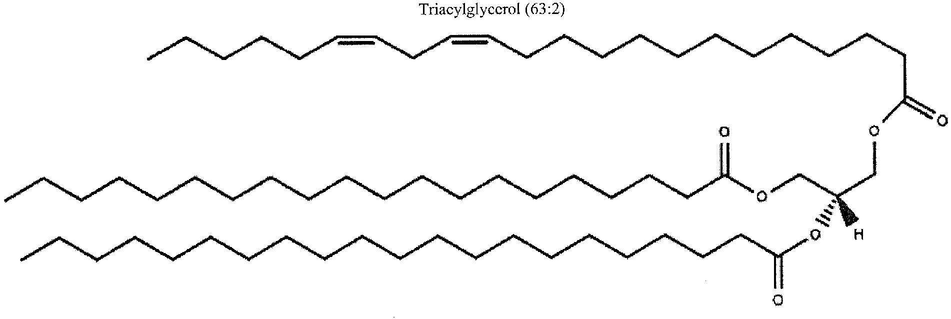 | |
| 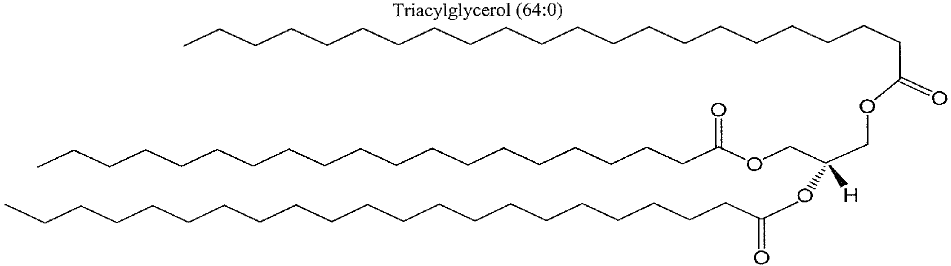 | |
| 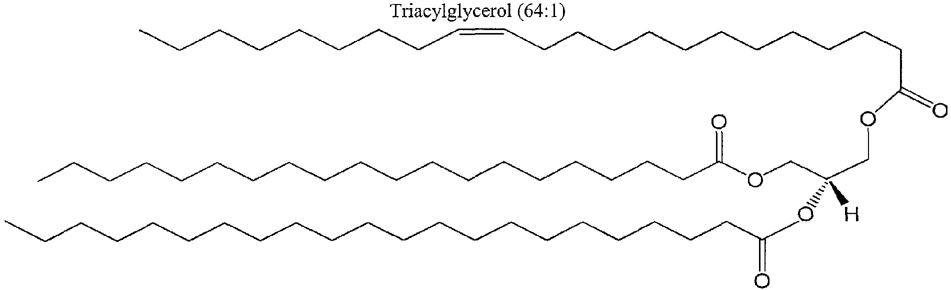 | |
| 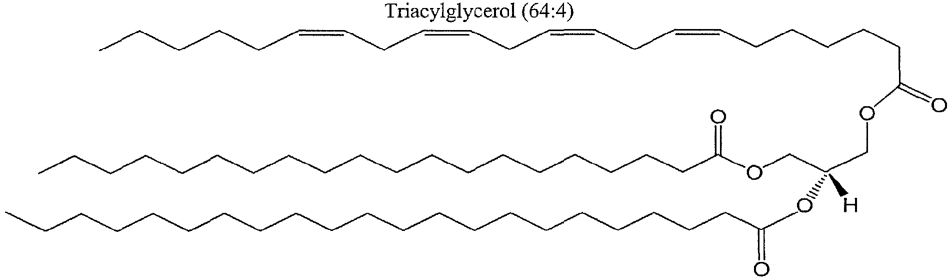 | |
| Triacylglycerol(64:5) C₆₇H₁₀₀O₆ | |
| Triacylglycerol(64:8) C₆₇H₁₁₄O₆ | |
| Triacylglycerol (65:1) C₆₈H₁₃₀O₆ | |
| Triacylglycerol (65:2) C₆₈H_{I28}O₆ | |
| Triacylglycerol (66:0) | |
| 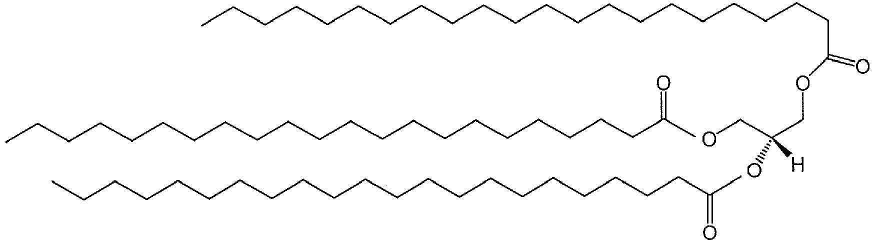 | |
| 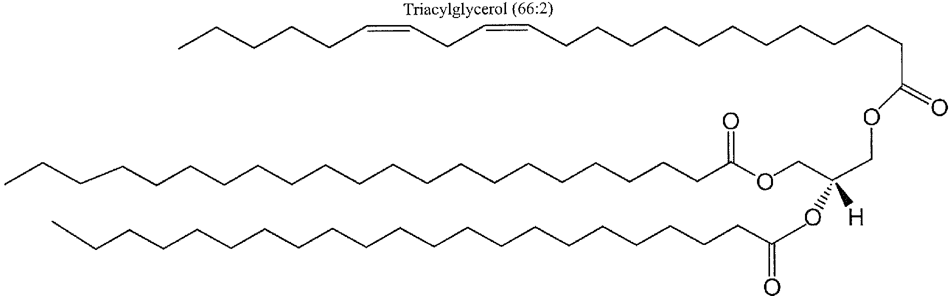 | |
| 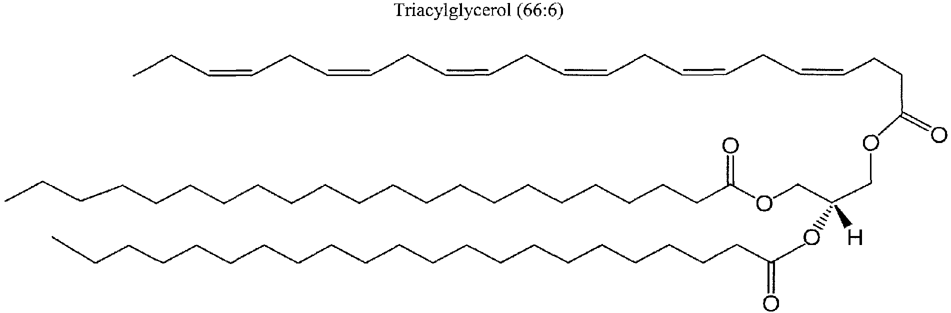 | |

### • Markers defined in Table 4

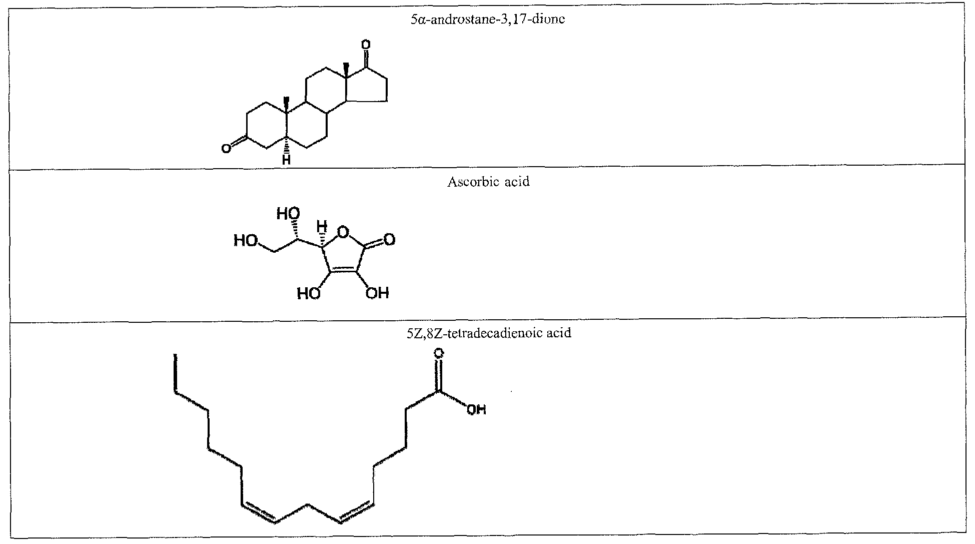

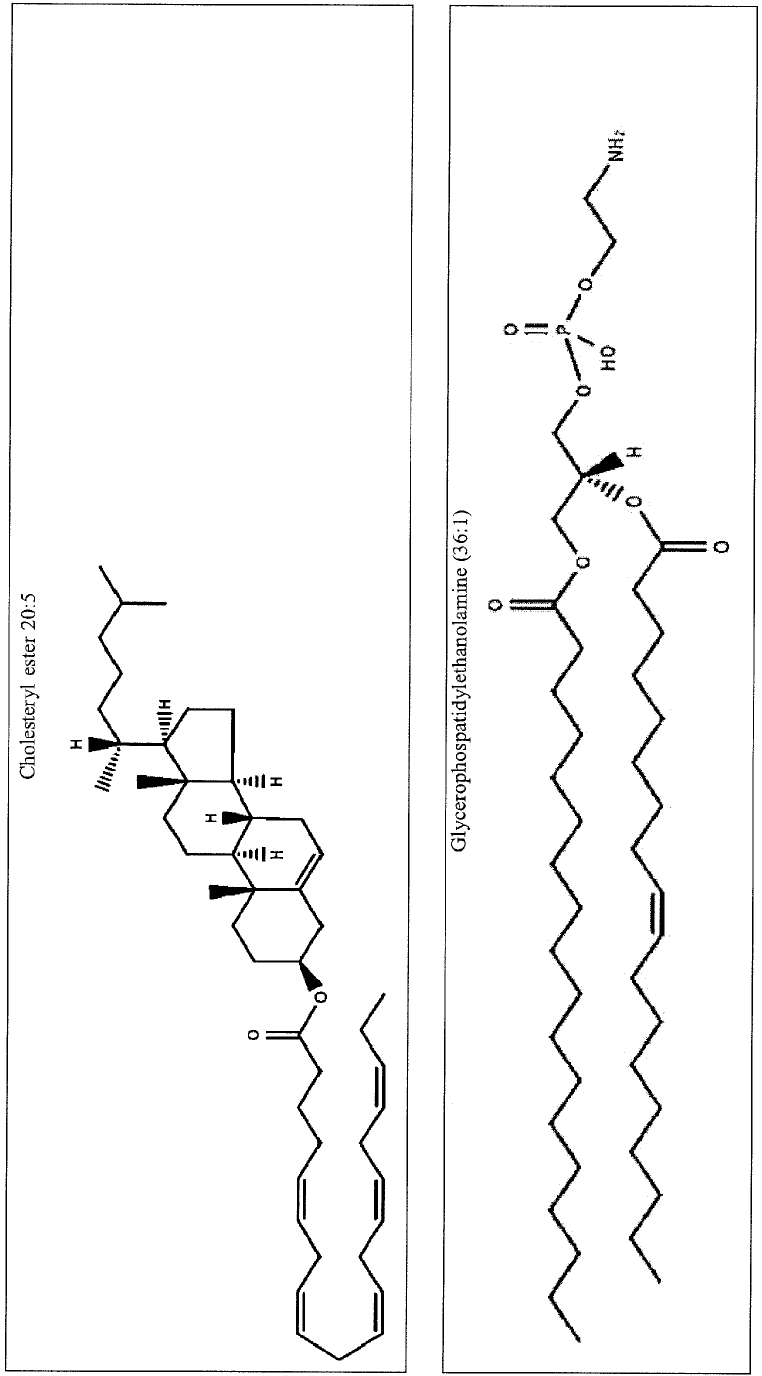

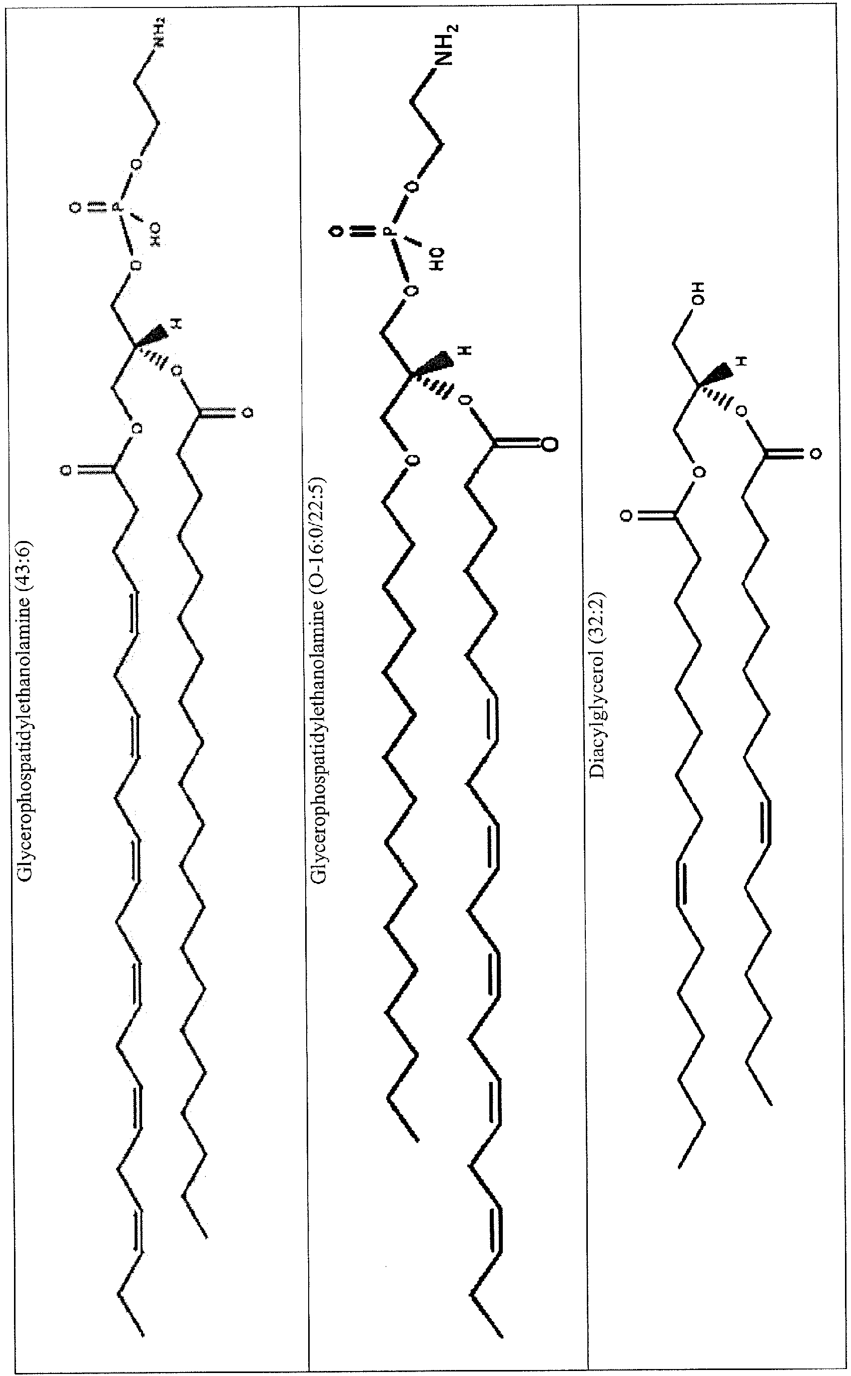

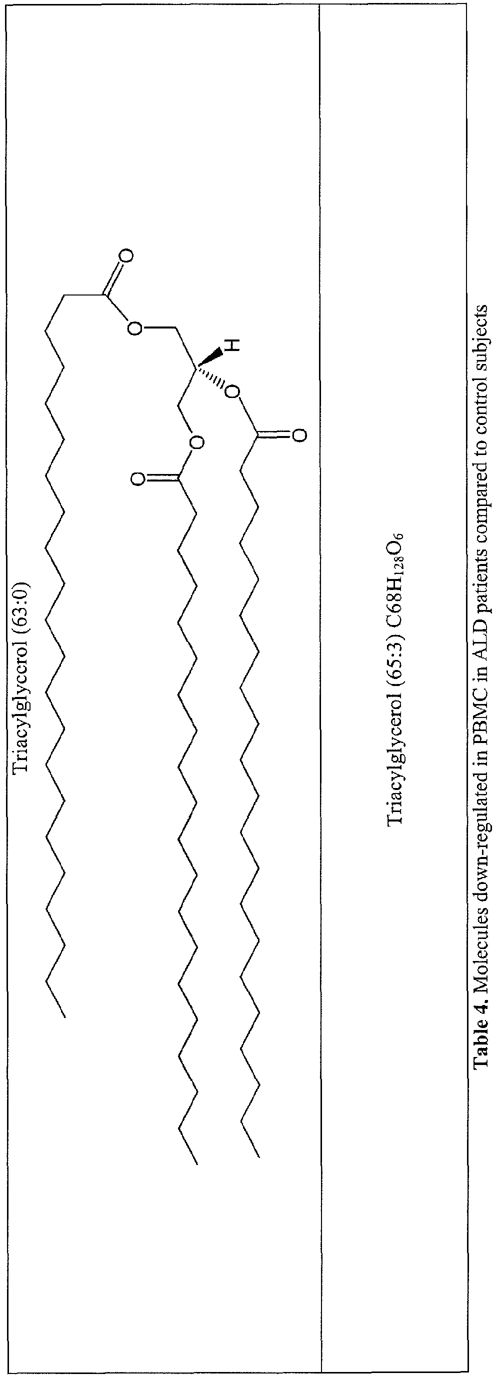

### • Markers defined in Table 5

**Table 5. Genes up-regulated in PBMC in ALD patients compared to control subjects**

| **Marker (Gene symbol)** | **Human GenBank Gene ID (release date)** | **Human UniProtKB/Swiss-Prot Accession num. (release date)** |
|---|---|---|
| Retinol dehydrogenase (RDH11) | 51109 (4 May 2015) | Q8TC12 (29 April 2015) |
| Sterol 26 hydroxilase (CYP27A1) | 1593 (12 May 2015) | Q02318 (29 April 2015) |
| Peroxisome proliferator-activated receptor delta (PPARβ/δ) | 5467 (17 May 2015) | Q03181 (29 April 2015) |

### Markers defined in Table 6

**Table 6. Plasma markers up-regulated in in ALD patients compared to control subjects**

| **Compound** | **Structure** |
|---|---|
| 12-Hydroxyeicosatetraenoic acid (12S-HETE) | 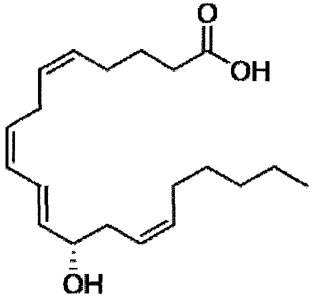 |
| 15-Hydroxyeicosatetraenoic acid (15S-HETE) | 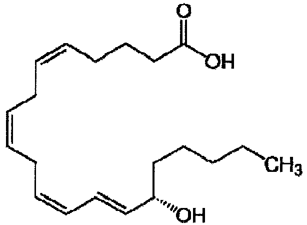 |
| Thromboxane B2 (TXB2) | 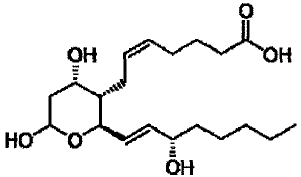 |

### • Markers defined in Table 7

**Table 7. Serum molecules up-regulated in ALD patients compared to control subjects**

| **Marker (Gene Symbol)** | **Human GenBank Gene ID (Release date)** | **Human UniProtKB/Swiss-Prot Accession num. (Release date)** |
|---|---|---|
| Hepatocyte growth factor (*HGF*) | 3082 (17 May 2015) | P14210 (29 April 2015) |
| Interleukin 6 (*IL6*) | 3569 (18 May 2015) | P05231 (29 April 2015) |
| Interleukin 8 (*IL8*) | 3576 (17 May 2015) | P10145 (29 April 2015) |
| Monocyte chemoattractant protein-1 or c-c motif chemokine 2 (*CCL*2 or *MCP-*1) | 6347 (17 May 2015) | P13500 (29 April 2015) |
| Tumor necrosis factor A *(TNFA)* | 7124 (17 May 2015) | P01375 (29 April 2015) |

### • Markers defined in Table 8

**Table 8. Cytokines, chemokines and receptors up-regulated in PBMC from AMN patients compared to control subjects**

| **Marker (Gene Symbol)** | **Human GenBank Gene ID (Release date)** | **Human UniProtKB/Swiss-Prot Accession num. (Release date)** |
|---|---|---|
| Complement component 5 (*C5*) | 727 | P01031 |
| | (17 May 2015) | (29 April 2015) |
| chemokine (C-X-C motif) | 6373 | 014625 |
| ligand 11 (*CXCL11*) | (3 May 2015) | (29 April 2015) |
| chemokine (C-X-C motif) | 6374 | P42830 |
| ligand 5 (CXCL5) | (12 May 2015) | (4 May 2015) |
| chemokine (C-X-C motif) | 6372 | P80162 |
| ligand 6 (CXCL6) | (4 May 2015) | (29 April 2015) |
| chemokine (C-X-C motif) | 4283 | Q07325 |
| ligand 9 (CXCL9) | (3 May 2015) | (29 April 2015) |
| chemokine (C-C) motif | 1235 | P51684 |
| receptor 6 (CCR6) | (4 May 2015) | (29 April 2015) |
| chemokine (C-X-C motif) | 3577 | P25024 |
| receptor 1 (CXCR1) | (4 May 2015) | (29 April 2015) |
| chemokine (C-X-C motif) | 6359 | Q16663 |
| ligand 5 (CCL15) | (17 May 2015) | (24 June 2015) |
| chemokine (C-X-C motif) | 6360 | 015467 |
| ligand 16 (CCL16) | (4 May 2015) | (29 April 2015) |
| chemokine (C-X-C motif) | 6366 | 000585 |
| ligand 21(CCL21) | (17 May 2015) | (29 April 2015) |
| chemokine (C-X-C motif) | 10563 | 043927 |
| ligand 21(CXCL13) | (3 May 2015) | (29 April 2015) |
| Interferon alpha-2 (IFNA2) | 3440 | P011563 |
| | (3 May 2015) | (29 April 2015) |
| Interleukin-36 alpha (IL36A) | 27179 | Q9UHA7 |
| | (4 May 2015) | (29 April 2015) |
| Interleukin-36 beta (IL36B) | 27177 | Q9NZH7 |
| | (4 May 2015) | (4 February 2015) |
| Interleukin-36 gamma (IL36G) | 56300 | Q9NZH8 |
| | (4 May 2015) | (29 April 2015) |
| Interleukin-22 (IL22) | 50616 | Q9GZX6 |
| | (17 May 2015) | (29 April 2015) |
| Lymphotoxin-beta (LTB) | 4050 | Q06646 |
| | (12 May 2015) | (29 April 2015) |
| Aminoacyl tRNA synthase complex-interacting multifunctional protein 1 (AIMP1) | 9255 | Q12904 |
| | (17 May 2015) | (29 April 2015) |
| C reactive protein (CRP) | 1401 | P02741 |
| | (17 Mav 2015 | (29 April 2015) |
| Chemokine (C-X-C motif) | 3579 | P25025 |
| receptor 2 (CXCR2) | (17 May 2015) | (29 April 2015) |
| Leukotriene B4 receptor (LTB4R) | 1241 | Q15722 |
| | (17 May 2015) | (29 April 2015) |
| chemokine (C-X-C motif) | 9547 | 095715 |
| ligand 14 (CXCL14) | (17 May 2015) | (29 April 2015) |
| Caspase recruitment domain familiy, member 18 (CARD18) | 59082 | P57730 |
| | (4 May 2015) | (29 April 2015) |
| Toll interacting protein (TOLLIP) | 54472 | Q6FIE9 |
| | (4 May 2015) | (29 April 2015) |
| Interleukin 10 (IL10) | 3586 | P22301 |
| | (17 May 2015) | (29 April 2015) |
| Interleukin 13 (IL13) | 3596 | P35225 |
| | (4 May 2015) | (29 April 2015) |
| Interleukin 17C (IL17C) | 27189 | Q9POM4 |
| | (12 May 2015) | (29 April 2015) |
| Interleukin 36 receptor antagonist (IL36RN) | 26525 | Q9UBH0 |
| | (4 May 2015) | (29 April 2015) |
| Interleukin 37 (IL37) | 27178 | Q9NZH6 |
| | (17 May 2015) | (29 April 2015) |
| Interleukin 5 (IL5) | 3567 | P05113 |
| | (10 May 2015) | (29 April 2015) |
| Interleukin 5 receptor alpha (IL5RA) | 3568 | Q01344 |
| | (12 May 2015) | (29 April 2015) |
| Interleukin 9 (IL9) | 3578 | P15248 |
| | (12 May 2015) | (29 April 2015) |
| Interleukin 9 receptor (IL9R) | 3581 | Q01113 |
| | (10 Mav 2015) | (29 April 2015) |
| Chemokine (C-C motif) | 6356 | P51671 |
| ligand 11 (CCL11) | (10 May 2015) | (29 April 2015) |
| Chemokine (C-C motif) | 6357 | Q99616 |
| ligand 13 (CCL13) | (4 May 2015) | (29 April 2015) |
| Chemokine (C-C motif) | 6361 | Q92583 |
| ligand 17 (CCL17) | (12 May 2015) | (29 April 2015) |
| Chemokine (C-C motif) | 6363 | Q99731 |
| ligand 19 (CCL19) | (17 May 2015) | (29 April 2015) |
| Chemokine (C-C motif) | 10344 | Q9Y258 |
| ligand 26 (CCL26) | (17 May 2015) | (29 April 2015) |
| Chemokine (C-C motif) | 6354 | P80098 |
| ligand 7 (CCL7) | (12 May 2015) | (29 April 2015) |
| Chemokine (C-C motif) | 6355 | P80075 |
| ligand 8 (CCL8) | (4 Mav 2015) | (29 April 2015) |
| Chemokine (C-X-C motif) | 6387 | P48061 |
| ligand 12 (CXCL12) | (17 Mav 2015) | (29 April 2015) |
| Chemokine (C-C motif) | 1232 | P51677 |
| receptor 3 (CCR3) | (12 May 2015) | (29 April 2015) |
| Chemokine (C-C motif) | 1237 | P51685 |
| receptor 8 (CCR8) | (4 May 2015) | (29 April 2015) |
| Signal transducer and activator | 6772 | P42224 |
| of transcription 1 (STAT1) | (17 May 2015) | (29 April 2015) |
| Interleukin 4 (IL4) | 3565 | P05112 |
| | (17 May 2015) | (29 April 2015) |
| Signal transducer and activator of transcription 6 (STAT6) | 6778 | P42226 |
| | (12 May 2015) | (29 April 2015) |
| Suppressor of cytokine | 9021 | 014543 |
| signaling 3 (SOCS3) | (4 May 2015) | (29 April 2015) |

In a particular embodiment, the method for the diagnosis of the invention comprises determining in a sample from a subject the levels of sphingosine-1-phosphate. In another particular embodiment, the diagnostic method of the disclosure comprises determining in a sample from a subject the levels of any combination of the markers from the group consisting of:
- sphingosine-1-phosphate,
- sphingosine kinase 2,
- sphingosine-1-phosphate receptor,
- adiponectin,
- neopterin
- the markers defined in Table 1,
- the markers defined in Table 2,
- the markers defined in Table 3,
- the markers defined in Table 4,
- the markers defined in Table 5,
- the markers defined in Table 6,
- the markers defined in Table 7 and
- the markers defined in Table 8.

In a particular embodiment, the diagnostic method of the disclosure comprises determining in a sample from a subject the levels of TAG(63:2).

In a particular embodiment, the diagnostic method of the invention comprises determining in a sample from a subject the levels of sphingosine-1-phosphate and TAG(63:2).

In a particular embodiment, the diagnostic method of the disclosure comprises determining in a sample from a subject the levels of 12-S-HETE and adiponectin.

In a particular embodiment, the diagnostic method of the disclosure comprises determining in a sample from a subject the levels of 15-S-HETE.In a particular embodiment, the diagnostic method of the invention comprises determining in a sample from a subject the levels of MCP-1.

In a particular embodiment, the diagnostic method of the disclosure comprises determining in a sample from a subject a value selected from the levels of at least one marker as defined in Table 7 and the expression levels of adiponectin. In a more particular embodiment, the diagnostic method of the disclosure comprises determining in a sample from a subject the levels of the markers as defined in Table 7 and the expression level of adiponectin.

In another particular embodiment, the diagnostic method of the disclosure comprises determining in a sample from a subject the levels of at least one marker as defined in Table 6. In a more particular embodiment, the diagnostic method of the disclosure comprises determining in a sample from a subject the levels of the markers as defined in Table 6.

In another particular embodiment, the diagnostic method of the disclosure comprises determining in a sample from a subject the expression levels of at least one marker selected from IL9, IL9R, IL4, IL5, IL5RA, IL10, CCL11, CCL13, CCL19, CCL26, CXCL12 and CCR8. In a more particular embodiment, the diagnostic method of the disclosure comprises determining in a sample from a subject the expression levels of IL9, IL9R, IL4, IL5, IL5RA, IL10, CCL11, CCL13, CCL19, CCL26, CXCL12 and CCR8.

In another particular embodiment, the diagnostic method of the disclosure comprises determining in a sample from a subject the expression levels of at least one marker selected from IL36A, IL36B, IL36G, IL36RN, IFNA2, CXCL11, CXCL6, CXCR1, CCL15, CCL16, CCL21, CXCL13, CXCL14 and CARD18. In a more particular embodiment, the diagnostic method of the disclosure comprises determining in a sample from a subject the expression levels of IL36A, IL36B, IL36G, IL36RN, IFNA2, CXCL11, CXCL6, CXCR1, CCL15, CCL16, CCL21, CXCL13, CXCL14 and CARD18.

The term "level", as used herein, refers to the quantity of a biomarker detectable in a sample.

The term "expression level", as used herein, refers to a measurable quantity of a gene product produced by the gene in a sample of the subject, wherein the gene product can be a transcriptional product or a translational product. As understood by the person skilled in the art, the gene expression level can be quantified by measuring the messenger RNA levels of said gene or of the protein encoded by said gene.

The methods for determining the level of a marker according to the diagnostic method of the disclosure will depend of the type or marker, namely, lipids, polar metabolites or genes.

When the marker according to the diagnostic method of the invention is a lipid, as defined in the appended claims, the level of marker can be determined by any method known in the art suitable for the determination and quantification of a lipid in a sample. By way of a non-limiting illustration, the level of a particular lipid can be determined by means of chromatography, mass spectrometry, nuclear resonance spectroscopy, fluorescence spectroscopy or dual polarization interferometry, a high performance separation method such as HPLC and/or an immunological method. In a particular embodiment, when the marker according to the first method of the invention is a lipid, the level of said biomarker id determined by means of a separation technique coupled to a method for identification and quantification of the lipid marker. In a more particular embodiment, the separation technique is an extraction with chloroform:methanol, and the method for identification and quantification of the lipid biomarker is chromatography, preferably RPLC, coupled to mass spectrometry, preferably QqTOF.

The term "RPLC" or "reversed-phase liquid chromatography", as used herein, refers to a technique to separate, identify and quantify the components of a mixture by using a polar mobile phase and a non-polar stationary phase.

The term "MS" or "mass-spectrometry", as used herein, refers to various methods such as tandem mass spectrometry, matrix assisted laser desorption ionization (MALDI), time-of-flight (TOF) mass spectrometry, MALDI-TOF-TOF mass spectrometry, MALDI Quadrupole-time-of-flight (Q-TOF) mass spectrometry, electrospray ionization (ESI)-TOF mass spectrometry, ESI-Q-TOF, ESI-TOF-TOF, ESI-ion trap mass spectrometry, ESI Triple quadrupole mass spectrometry, ESI Fourier Transform mass spectrometry (FTMS), MALDI-FTMS, MALDI-Ion Trap-TOF, and ESI-Ion Trap TOF. These mass spectrometry methods are well known in the art. At its most basic level, mass spectrometry involves ionizing a molecule and then measuring the mass of the resulting ion. Since molecules ionize in a way that is well known, the molecular weight of the molecule can generally be accurately determined from the mass of the ion. MSⁿ refers to subsequent fragmentation of ions obtained in order to further ensure the identity of the measured ion. For instance, MS² or tandem mass spectrometry (MS/MS) may be used to identify proteins because it can provide information in addition to parent ion molecular weight. Tandem mass spectrometry involves first obtaining a mass spectrum of the ion of interest (parent ions), then fragmenting that ion and obtaining a mass spectrum of the fragments (product ions). Quantifying the amount of product ions derived from a specific parent ion is termed transition. Tandem mass spectrometry thus provides both molecular weight information and a fragmentation pattern that can be used in combination along with the molecular weight information to identify the exact sequence of a peptide or protein or the chemical structure of characterized metabolites. In MS³ for instance, fragmenting the obtained product ions (i.e. converting them in parent ions) would offer a novel set of product ions, useful for quantification and/or characterization. The term "ESI-Q-TOF" or "electrospray quadrupole-time-of-flight", as used herein, refers to a mass spectrometry technique, consisting on the use of an instrument or mass spectrometer in which the mass-to-charge ratio of the specific ions are determined by measurement of the time they take travelling from the first quadrupole to the detector. The ions are produced from the chromatography eluent by applying a known source of electric field, under a current of gas (usually nitrogen) at determined conditions. This procedure of ionization is termed electro spray. The obtained ions are introduced into a high vacuum system and then the first quadrupole accelerates the ions by exposing them to electric fields adjusted to a certain level. After this, all the ions with the same charge will exhibit the same kinetic energy. Therefore the velocity of the ion in the high vacuum of the instrument will depend on the mass-to-charge ratio. Being the distance known, and measuring the "time-of-flight" of these ions, one could estimate the mass-to-charge ratio, based on experimental substances with known mass-to-charge ratios submitted to the same conditions. The term "ESI-QqQ" or "Electrospray triple quadrupole" refers to a mass spectrometry technique, which consists of a similar mass spectrometry system where the sequential use of three quadrupoles allows for the selection of specific mass spectrometric transitions, as defined above.

When the marker according to the diagnostic method of the disclosure is a polar metabolite, the level of said marker can be determined by any method known in the art suitable for the determination and quantification of polar metabolites in a sample. By way of a non-limiting illustration, the level of a particular polar metabolite can be determined by means of chromatography, mass spectrometry, nuclear resonance spectroscopy, fluorescence spectroscopy or dual polarization interferometry, a high performance separation method such as HPLC and/or an immunological method. In a particular embodiment, when the marker according to the diagnostic method of the invention is a polar metabolite, the level of said marker is determined by means of a separation technique coupled to a method for identification and quantification of the polar metabolite. In a more particular embodiment, the separation technique is an extraction with methanol, and the method for identification and quantification of the polar metabolite is chromatography, preferably HPLC, coupled to mass spectrometry, preferably ESI-Q-TOF or ESI-QqQ or similar MSⁿ (MS/MS or MS/MS/MS) techniques.

The term "HPLC" or "high performance liquid chromatography" as used herein, refers to a technique used in analytic chemistry to separate, identify and quantify the components of a mixture in which the degree of separation is increased by forcing a mobile phase under pressure through a stationary phase on a support matrix, typically a densely packed column.

When the marker according to the diagnostic method of the invention is a gene, as defined in the appended claims, the expression levels of said gene can be determined by measuring the messenger RNA levels of said gene or the levels of the protein encoded by said gene.

The level of a messenger RNA can be determined by methods well known in the art. For example the nucleic acid contained in the is first extracted according to standard methods, for example using lytic enzymes or chemical solutions or extracted by nucleic-acid-binding resins following the manufacturer's instructions. The extracted mRNA is then detected by hybridization (e.g., Northern blot analysis or by oligonucleotide microarrays after converting the mRNA into a labeled cDNA) and/or amplification (e.g., RT-PCR). Quantitative or semi-quantitative RT-PCR is preferred. Real-time quantitative or semiquantitative RT-PCR is particularly advantageous. Preferably, primer pairs were designed in order to overlap an intron, so as to distinguish cDNA amplification from putative genomic contamination. Suitable primers may be easily designed by the skilled person. Other methods of amplification include ligase chain reaction (LCR), transcription-mediated amplification (TMA), strand displacement amplification (SDA) and nucleic acid sequence based amplification (NASBA). Preferably, the quantity of mRNA is measured by quantitative or semi-quantitative RT-PCR or by real-time quantitative or semi-quantitative RT-PCR.

In a particular embodiment, when the marker according to the diagnostic method of the invention is sphingosine kinase 2 and/or sphingosine-1-phosphate receptor, the expression levels of said markers are determined by measuring the levels of their respective mRNAs.

The level of a protein can be determined by any method known in the art suitable for the determination and quantification of a protein in a sample. By way of a non-limiting illustration, the level of a protein can be determined by means of a technique which comprises the use of antibodies with the capacity for binding specifically to the assayed protein (or to fragments thereof containing the antigenic determinants) and subsequent quantification of the resulting antigen-antibody complexes, or alternatively by means of a technique which does not comprise the use of antibodies such as, for example, by techniques based on mass spectroscopy. The antibodies can be monoclonal, polyclonal or fragment thereof, Fv, Fab, Fab' and F(ab')2, scFv, diabodies, triabodies, tetrabodies and humanized antibodies. Similarly, the antibodies may be labeled. Illustrative, but non-exclusive, examples of markers that can be herein used include radioactive isotopes, enzymes, fluorophores, chemoluminescent reagents, enzyme cofactors or substrates, enzyme inhibitors, particles, or dyes. There is a wide variety of known test that can be used according to the present invention, such as combined application of non-labeled antibodies (primary antibodies) and labeled antibodies (secondary antibodies), Western blot or immunoblot, ELISA (enzyme-linked immunosorbent assay), RIA (radioimmunoassay), competitive EIA (enzyme immunoassay), DAS-ELISA (double antibody sandwich ELISA), two-dimensional gel electrophoresis, capillary electrophoresis, immunocytochemical and immunohistochemical techniques, immunoturbidimetry, immunofluorescence, techniques based on the use of biochips or protein microarrays including specific antibodies or assays based on the colloidal precipitation in formats such as reagent strips and assays based on antibody-linked quantum dots. Other forms of detecting and quantifying proteins include, for instance, affinity chromatography techniques or ligand-binding assays.

The diagnostic method of the invention comprises/involves comparing the level of the markers with a reference value.

The term "reference value", as used herein, relates to a predetermined criteria used as a reference for evaluating the values or data obtained from the samples collected from a subject. The reference value or reference level can be an absolute value, a relative value, a value that has an upper or a lower limit, a range of values, an average value, a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

The reference value according to the first method of the invention can be obtained from one or more subjects who do not suffer from ALD (i.e., control subjects). A subject is considered to not suffer from ALD if they have not been diagnosed with ALD. The diagnosis of ALD can be made based on the following criteria. If ALD is suspected in a male, ALD diagnostic is made when increased VLCFA (very long chain fatty acid) levels are detected in plasma, and confirmed when identification of a mutation in the ABCD1 gene is achieved. If ALD is suspected in a female, the diagnostic test of choice is mutational analysis of the *ABCD1* gene since 15% of women with ALD have normal plasma VLCFA levels.

According to the diagnostic method of the invention, the level or the expression level of a marker is considered "decreased" when the level/expression level of said marker in a sample is lower than its reference value. The level/expression level of a marker is considered to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

Likewise, in the context of the diagnostic method of the invention, the level or the expression level of a marker is considered "increased" when the level/expression level of said marker in a sample is higher than its reference value. The level/expression level of a biomarker is considered to be higher than its reference value when it is at least 1.5%, at least 2%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value.

### Method for monitoring the progression of an adrenoleukodystrophy

In a second aspect, the invention relates to a method for monitoring the progression of an adrenoleukodystrophy in a subject suffering from adrenoleukodystrophy, hereinafter second method of the invention, comprising determining in a sample from said subject a value selected from the group consisting of
- the levels of sphingosine-1-phosphate,
- the expression levels of sphingosine kinase 2,
- the expression levels sphingosine-1-phosphate receptor,
wherein
increased levels of sphingosine-1-phosphate,
increased levels of sphingosine kinase 2,
increased levels of sphingosine-1-phosphate receptor,
with respect to a value determined in a sample from said subject at an earlier time point is indicative of a worsening of the adrenoleukodystrophy or wherein
decreased levels of sphingosine-1-phosphate,
decreased levels of sphingosine kinase 2,
decreased levels of sphingosine-1-phosphate receptor,
with respect to a value determined in a sample from said subject at an earlier time point is indicative of an amelioration of the adrenoleukodystrophy.

The terms "adrenoleukodystrophy", "sample", "level", "expression level", "marker", "increased" and "decreased" have been defined in connection with the first method of the invention. The markers sphingosine-1-phosphate, sphingosine kinase 2, sphingosine-1-phosphate receptor, adiponectin, neopterin and the markers defined in Tables 1-8, as well as the techniques for determining the level of these markers, have also been defined in connection to the first method of the invention. The particular and preferred embodiments of the first method of the invention regarding these terms are also applicable to the second method of the invention.

In a particular embodiment, the second method of the invention comprises determining in a sample from a subject the levels of sphingosine-1-phosphate. In another particular embodiment, the second method of the disclosure comprises determining in a sample from a subject the levels of any combination of the markers from the group consisting of:
- sphingosine-1-phosphate,
- sphingosine kinase 2,
- sphingosine-1-phosphate receptor,
- adiponectin,
- neopterin,
- the markers defined in Table 1,
- the markers defined in Table 2,
- the markers defined in Table 3,
- the markers defined in Table 4,
- the markers defined in Table 5,
- the markers defined in Table 6,
- the markers defined in Table 7 and
- the markers defined in Table 8.

In a particular embodiment, the second method of the disclosure comprises determining in a sample from a subject the levels of TAG(63:2).

In a particular embodiment, the second method of the invention comprises determining in a sample from a subject the levels of sphingosine-1-phosphate and TAG(63:2).

In a particular embodiment, the second method of the disclosure comprises determining in a sample from a subject the levels of 12-S-HETE and adiponectin.

In a particular embodiment, the second method of the disclosure comprises determining in a sample from a subject the levels of 15-S-HETE.In a particular embodiment, second method of the invention comprises determining in a sample from a subject the levels of MCP-1.

In a particular embodiment, second method of the disclosure comprises determining in a sample from a subject a value selected from the levels of at least one marker as defined in Table 7 and the expression levels of adiponectin. In a more particular embodiment, the second method of the disclosure comprises determining in a sample from a subject the levels of the markers as defined in Table 7 and the expression level of adiponectin.

In another particular embodiment, the second method of the disclosure comprises determining in a sample from a subject the levels of at least one marker as defined in Table 6. In a more particular embodiment, the second method of the disclosure comprises determining in a sample from a subject the levels of the markers as defined in Table 6.

In another particular embodiment, the second method of the disclosure comprises determining in a sample from a subject the expression levels of at least one marker selected from IL9, IL9R, IL4, IL5, IL5RA, IL10, CCL11, CCL13, CCL19, CCL26, CXCL12 and CCR8. In a more particular embodiment, the second method of the disclosure comprises determining in a sample from a subject the expression levels of IL9, IL9R, IL4, IL5, IL5RA, IL10, CCL11, CCL13, CCL19, CCL26, CXCL12 and CCR8.

In another particular embodiment, the second method of the disclosure comprises determining in a sample from a subject the expression levels of at least one marker selected from IL36A, IL36B, IL36G, IL36RN, IFNA2, CXCL11, CXCL6, CXCR1, CCL15, CCL16, CCL21, CXCL13, CXCL14 and CARD18. In a more particular embodiment, the second method of the disclosure comprises determining in a sample from a subject the expression levels of IL36A, IL36B, IL36G, IL36RN, IFNA2, CXCL11, CXCL6, CXCR1, CCL15, CCL16, CCL21, CXCL13, CXCL14 and CARD 18.

In a particular embodiment, the adrenoleukodystrophy is selected from the group consisting of adult adrenomyeloneuropathy (AMN), cerebral adrenomyeloneuropathy (cAMN) and the childhood variant of adrenoleukodystrophy (cALD).

The ALD can occur with or without inflammatory demyelination. In a particular embodiment, the adrenoleukodystrophy occurs without inflammatory demyelination.

In a particular embodiment, when the marker according to the second method of the invention is sphingosine kinase 2 and/or sphingosine-1-phosphate receptor, the expression levels of said markers are determined by measuring the levels of their respective mRNAs.

In a more particular embodiment, the sample is a sample containing PBMC. In an even more particular embodiment, the sample containing PBMC is blood.

The term "subject suffering from ALD", as used herein, refers to a subject who has been diagnosed with ALD. The diagnosis of ALD can be made based on the diagnosis criteria explained in connection with the diagnostic method of the invention.

The term "monitoring the progression", as used herein, refers to the determination of the evolution of the disease in a subject diagnosed with ALD, i.e., whether the ALD is worsening or whether it is ameliorating.

The term "worsening of the ALD", as used herein, means that the disease is progressing to a later stage with respect to the stage at the first time point measured, for instance, worsening of locomotor capacities or increased spasticity. In a particular embodiment, a worsening of the ALD means the appearance of cerebral inflammatory demyelination.. In another particular embodiment, a worsening of the ALD means a progression from AMN to cAMN.

The term "amelioration", as used herein, refers to the reduction of one or more symptoms of a disorder, condition, or disease, for example ALD.

The second method of the invention involves comparing the levels or expression levels of one or more markers in a sample from a subject with a value, i.e., the levels or expression levels of the same marker or markers, determined in a sample from said subject at an earlier time point. In a particular embodiment, the level/expression level of the marker in a sample is compared with the level/expression level of said marker in the same type of sample.

### Method for monitoring the effect of an adrenoleukodystrophy therapy

In a third aspect, the invention relates to a method for monitoring the effect of an adrenoleukodystrophy therapy in a subject suffering from adrenoleukodystrophy, hereinafter third method of the invention, comprising determining in a sample from said subject a value selected from the group consisting of
- the levels of sphingosine-1-phosphate,
- the expression levels of sphingosine kinase 2,
- the expression levels sphingosine-1-phosphate receptor,
wherein
increased levels of sphingosine-1-phosphate,
increased levels of sphingosine kinase 2,
increased levels of sphingosine-1-phosphate receptor,
with respect to the value determined prior to the administration of the therapy is indicative that the therapy is not effective
or wherein
decreased levels of sphingosine-1-phosphate,
decreased levels of sphingosine kinase 2,
decreased levels of sphingosine-1-phosphate receptor,
with respect to a value determined prior to the administration of the therapy is indicative that the therapy is effective.

The terms "adrenoleukodystrophy", "sample", "level", "expression level", "marker", "increased" and "decreased" have been defined in connection with the first method of the invention. The markers sphingosine-1-phosphate, sphingosine-2-phopshate kinase, sphingosine-1-phosphate receptor, adiponectin, neopterin and the markers defined in Tables 1-8, as well as the techniques for determining the level of these markers, have also been defined in connection to the first method of the invention defined in the appended claims.

The term "subject suffering from ALD" has been defined in connection with the second method of the invention defined in the appended claims. The particular and preferred embodiments of the first method and second method of the invention regarding these terms are also applicable to the third method of the invention defined in the appended claims.

In a particular embodiment, the adrenoleukodystrophy is selected from the group consisting of adult adrenomyeloneuropathy (AMN), cerebral adrenomyeloneuropathy (cAMN) and the childhood variant of adrenoleukodystrophy (cALD).

The ALD can occur with or without inflammatory demyelination. In a particular embodiment, the ALD occurs without inflammatory demyelination.

In a particular embodiment, the third method of the invention comprises determining in a sample from a subject the levels of sphingosine-1-phosphate. In another particular embodiment, the third method of the disclosure comprises determining in a sample from a subject the levels of any combination of the markers from the group consisting of:
- sphingosine-1-phosphate,
- sphingosine kinase 2,
- sphingosine-1-phosphate receptor,
- adiponectin,
- neopterin,
- the markers defined in Table 1,
- the markers defined in Table 2,
- the markers defined in Table 3,
- the markers defined in Table 4,
- the markers defined in Table 5,
- the markers defined in Table 6,
- the markers defined in Table 7 and
- the markers defined in Table 8.

In a particular embodiment, the third method of the disclosure comprises determining in a sample from a subject the levels of TAG(63:2).

In a particular embodiment, the third method of the disclosure comprises determining in a sample from a subject the levels of sphingosine-1-phosphate and TAG(63:2).

In a particular embodiment, the third method of the disclosure comprises determining in a sample from a subject the levels of 12-S-HETE and adiponectin.

In a particular embodiment, the third method of the disclosure comprises determining in a sample from a subject the levels of 15-S-HETE.In a particular embodiment, the third method of the disclosure comprises determining in a sample from a subject the levels of MCP-1.

In a particular embodiment, the third method of the disclosure comprises determining in a sample from a subject a value selected from the levels of at least one marker as defined in Table 7 and the expression levels of adiponectin. In a more particular embodiment, the third method of the disclosure comprises determining in a sample from a subject the levels of the markers as defined in Table 7 and the expression level of adiponectin.

In another particular embodiment, the third method of the disclosure comprises determining in a sample from a subject the levels of at least one marker as defined in Table 6. In a more particular embodiment, the third method of the disclosure comprises determining in a sample from a subject the levels of the markers as defined in Table 6.

In another particular embodiment, the third method of the disclosure comprises determining in a sample from a subject the expression levels of at least one marker selected from IL9, IL9R, IL4, IL5, IL5RA, IL10, CCL11, CCL13, CCL19, CCL26, CXCL12 and CCR8. In a more particular embodiment, the third method of the disclosure comprises determining in a sample from a subject the expression levels of IL9, IL9R, IL4, IL5, IL5RA, IL10, CCL11, CCL13, CCL19, CCL26, CXCL12 and CCR8.

In another particular embodiment, the third method of the disclosure comprises determining in a sample from a subject the expression levels of at least one marker selected from IL36A, IL36B, IL36G, IL36RN, IFNA2, CXCL11, CXCL6, CXCR1, CCL15, CCL16, CCL21, CXCL13, CXCL14 and CARD18. In a more particular embodiment, the third method of the disclosure comprises determining in a sample from a subject the expression levels of IL36A, IL36B, IL36G, IL36RN, IFNA2, CXCL11, CXCL6, CXCR1, CCL15, CCL16, CCL21, CXCL13, CXCL14 and CARD18.

In a particular embodiment, the marker according to the third method of the disclosure is selected from the group consisting of adiponectin, TNF, IL-8, 12S-HETE, 15S-HETE, TXB2, INFA2, IL4, IL10, IL36, CCR3, CXCL9 and neopterin.

In a particular embodiment, when the marker according to the third method of the invention is sphingosine kinase 2 and/or sphingosine-1-phosphate receptor, the expression levels of said markers are determined by measuring the levels of their respective mRNAs.

In a more particular embodiment, the sample is a sample containing PBMC. In an even more particular embodiment, the sample containing PBMC is blood.

The term "adrenoleukodystrophy therapy", as used herein, refers to the attempted remediation of a health problem, usually following a diagnosis, or to prevention or the appearance of a health problem. As such, it is not necessarily a cure, i.e. a complete reversion of a disease. Said therapy may or may not be known to have a positive effect on a particular disease. This term includes both therapeutic treatment and prophylactic or preventative measures, in which the object is to prevent or stop (reduce) an undesired physiological change or disorder, such as, cancer. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival, disease free survival and symptom free survival, in comparison with the expected survival if treatment is not received. Those subjects needing treatment include those subjects already suffering the condition or disorder, as well as those with the tendency to suffer the condition or disorder or those in which the condition or disorder must be prevented.

Illustrative non limitative examples of ALD therapies include: an antioxidant, an antioxidant targeted to mitochondria, a histone deacetylase inhibitor, an inhibitor of mitochondria transition pore opening, an anti-inflammatory drug, a PPAR agonist, a RXR agonist, a sirtuin 1 agonist, a hypolipidemic drug, a fatty acid composition able to decrease circulating levels of hexacosanoic acid (C26:0) and an autophagy activator

The term "antioxidants", as used herein, refer to substances that reduce the levels of reactive oxygen species, for instance preventing the formation of such reactive oxygen species or removing them before they produce any damage. Examples of antioxidants are alpha-lipoic acid, vitamin E, and N-acetylcysteine.

The term "antioxidants targeted to mitochondria", as used herein, refer to those antioxidants that are selectively concentrated within mitochondria *in vivo.* Examples of antioxidants targeted to mitochondria are mitoquinone (MitoQ) and [2-(3,4-dihydro-6-hydroxy-2,5, 7,8-tetramethyl-2H-1-benzopyran-2-yl)ethyl]triphenylphosphonium bromide (MitoVitE).

The term "histone deacetylase inhibitors", as used herein, refer to substances that interfere with the function of histone deacetylase. Examples of histone deacetylase inhibitors are vorinostat, romidepsin, panobinostat, valproic acid, belinostat, mocetinostat, PCI-24781, entinostat, SB939, reminostat, givinostat, CUDC-101, AR-42, CHR-2845, CHR-3996, 4SC-202, CG200745, ACY-1215, sulforaphane and kevetrin. "Inhibitors of mitochondria transition pore opening", as used herein, refer to substances that block the non-specific increase in the permeability of the inner membrane of the mitochondria, caused by the opening of an inner membrane channel. Examples of inhibitors of mitochondria transition pore opening are cyclosporin A and derivatives thereof, NIM811, 2-aminoethoxydiphenyl borate and bongkrekic acid.

The term "anti-inflammatory drugs", as used herein, refer to substances that reduce inflammation. Examples of anti-inflammatory drugs are salicylates, such as acetylsalicylic acid, diflunisal and salsalate; propionic acid derivatives, such as ibuprofen, naproxen, fenoprofen, ketoprofen, dexketoprofen, flurbiprofen, oxaprozin, loxoprofen; acetic acid derivatives, such as indomethacin, sulindac, etodolac, ketorolac, diclofenac, nabumetone; enolic acid derivatives, such as piroxicam, meloxicam, tenoxicam, droxicam, lornoxicam and isoxicam; fenamic acid derivatives such as mefenamic acid, meclofenamic acid, flufenamic acid, and tolfenamic acid; selective COX-2 inhibitors such as celecoxib, rofecoxib, valdecoxib, parecoxib, lumiracoxib, etoricoxib and firocoxib; sulphonanilides such as nimesulide; and other compounds such as licofelone.

The term "PPAR agonists", as used herein, refer to substances that stimulate the peroxisome proliferator-activated receptors. Examples of PPAR agonists are GW-9662, thiazolidinediones, such as rosiglitazone, pioglitazone and its derivatives; fibrates, such as bezafibrate, ciprofibrate, clofibrate, gemfibrozil and fenofibrate; and glitazars such as muraglitazar, tesaglitazar and aleglitazar.

The term "RXR agonists", as used herein, refer to substances that stimulate the retinoid X receptor. Examples of RXR agonists are CD 3254, docosahexaenoic acid, fluorobexarotene, bexarotene, retinoic acid and SR 11237.

The term "sirtuin 1 agonists", as used herein, refer to substances that stimulate the sirtuin 1 enzyme. Examples of sirtuin 1 agonists are resveratrol and SRT-1720.

The term "hypolipidemic agents", as used herein, refer to substances other than PPAR agonist and fibrates that lower the lipid low density lipoproteins (LDL) and/or increase the high density lipoprotein (HDL) in blood. Examples of hypolipidemic agents are statins, such as atorvastatin, cerivastatin, fluvastatin, lovastatin, mevastatin, pitavastatin, pravastatin, rosuvastatin, and simvastatin; niacin; bile acid sequestrants, such as cholestyramine, colesevelam and colestipol; other compounds such as phytosterols, ezetimibe, orlistat, and niacin.

The term "fatty acid composition able to decrease circulating levels of hexacosanoic acid (C26:0)" refers to a composition of fatty acids capable of producing a decrease in the circulating levels of hexacosanoic acid (C26:0), which can be determined by any suitable technique known by the skilled person. Illustrative non-limitative examples of said fatty acid composition is Lorenzo's oil, which is formed by 4 parts of glyceryl trioleate and 1 part glyceryl trierucate, which are the triacylglycerol forms of oleic acid and erucic acid and are prepared from olive oil and rapeseed oil.

In a particular embodiment, the ALD therapy comprises an antioxidant compound. In a more particular embodiment, the antioxidant compound is a combination of N-acetylcystein, lipoic acid and vitamin E.

### Use of fingolimod

In a fourth aspect, the invention relates to an inhibitor of sphingosine-1-phosphate receptor 1 for use in the treatment and/or prevention of an adrenoleukodystrophy.

Alternatively, the disclosure relates to the use of an inhibitor of sphingosine-1-phosphate receptor lin the manufacture of a medicament for the treatment and/or prevention of an adrenoleukodystrophy.

Alternatively, the disclosure relates to a method of treatment and/or prevention of an adrenoleukodystrophy in a subject comprising administering to said subject a therapeutically effective amount of an inhibitor of sphingosine-1-phosphate receptor 1.

The terms "adrenoleukodystrophy" and "subject" as well as theirs particular and preferred embodiments have been previously defined.

The term "inhibitor of sphingosine-1-phosphate receptor 1" or "inhibitor of S1PR1", as used herein, refers to a compound inhibiting the activity *in vivo* or *in vitro* of sphingosine-1-phosphate receptor 1, including, without limitation, antagonists, inhibitory antibodies, compounds which prevent the expression of the gene encoding the protein and expression and compounds which lead to reduced mRNA or protein levels. In a particular embodiment, the S1PR1 inhibitor is capable of producing the intracelullar degradation of the S1PR1. The term "sphingosine-1-phosphate receptor 1" or "S1PR1" has been previously defined.

The skilled person can determine if a particular compound is an inhibitor of S1PR1 by any suitable assay, for example, the assay described in EP2364976 A1 In a particular embodiment, a S1PR1 inhibitor is capable of inducing a detectable decrease in SP1 receptor activity in vivo *or in vitro* (for example, at least a 10% decrease in SP1 receptor activity as measured by an assay such as the assay described in EP2364976 A1). In a particular embodiment, a S1PR1 inhibitor is capable of diminishing the circulating levels of S1P. Circulating levels of S1P can be determined by mass spectrometry as previously explained. In another particular embodiment, an S1PR1 inhibitor is capable of diminishing the expression levels of S1PR1 in peripheral lymphocytes. The expression levels of S1PR1 can be determined by measuring the messenger RNA levels of said gene or the levels of the protein encoded by said gene, as previously explained.

Illustrative non-limitative examples of inhibitor of S1PR1 are the following: ozanimod, FTY720, AAL(R), KRP-203, ceralifimod, ponesimod, siponimod, CYM-5442, RP-001, BAF312, ONO-4641, CS-0777, RPC-1063, SEW2871, VPC2309, VPC4416, W146, VPC25239, GSK2018682, fingolimod, an analogue, metabolite or derivative thereof, or a pharmaceutically acceptable salt thereof.

In a particular embodiment, the inhibitor of sphingosine-1-phosphate receptor 1 is fingolimod, an analogue, metabolite or derivative thereof, or a pharmaceutically acceptable salt thereof.

The term "fingolimod" or "FTY720", as used herein, refers to the compound of formula

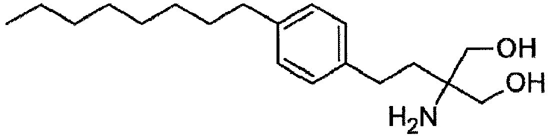

Fingolimod is an analogue of sphingosine-1 phosphate (S1P) and is phosphorylated by sphingosine kinase 2 (SPHK2) into fingolimod phosphate. Similar to S1P, fingolimod-phosphate is able to bind sphingosine-1 phosphate receptor 1 (S1PR1), which is then internalized. The S1P receptor is then degraded, therefore preventing cell surface signaling. Hence, fingolimod causes indirect antagonism of the S1P receptor's function.

The term "analogue", as used herein, refers to any entity structurally derived or homologous to a compound which has similar biochemical activity with respect to that compound. In a particular embodiment, the fingolimod analogue is able to bind a sphingosine 1 phosphate receptor, in particular S1PR1, and promote its intracellular degradation. Illustrative non-limitative examples of fingolimod analogues are: siponimod, KRP-203, ponesimod, RPC-1063 and the compounds described in the US patent No. 8,673,982. In a particular embodiment, the fingolimod analogue is selected from the group consisting of siponimod, KRP-203, ponesimod and RPC-1063. In a more particular embodiment, the fingolimod analogue is siponimod.

The term "siponimod" or "BAF312" refers to the compound of formula:

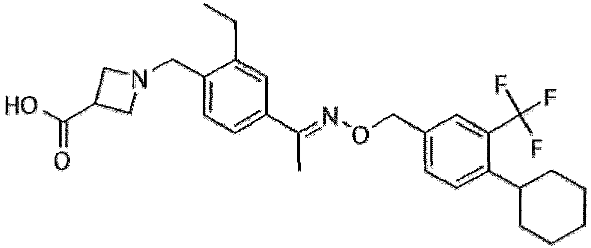

The term "KRP-203" refers to the compound of formula:

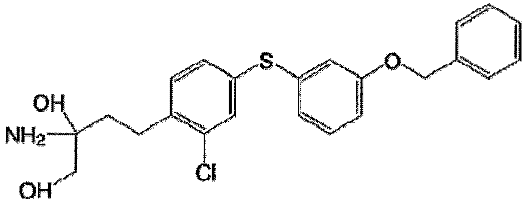

The term "ponesimod" refers to the compound of formula:

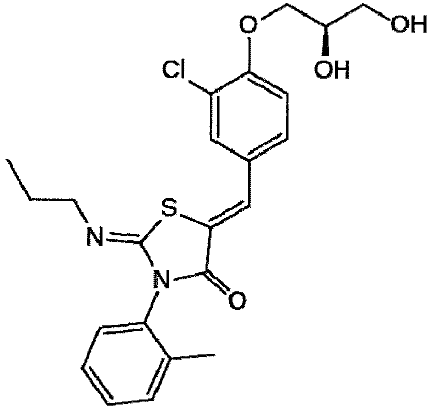

The term "RPC-1063" refers to the compound of formula 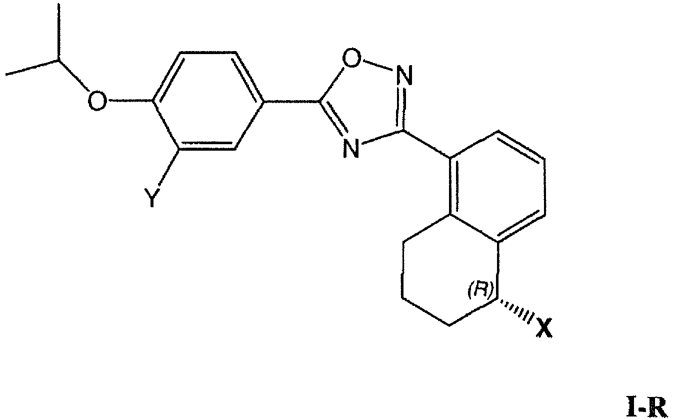 or

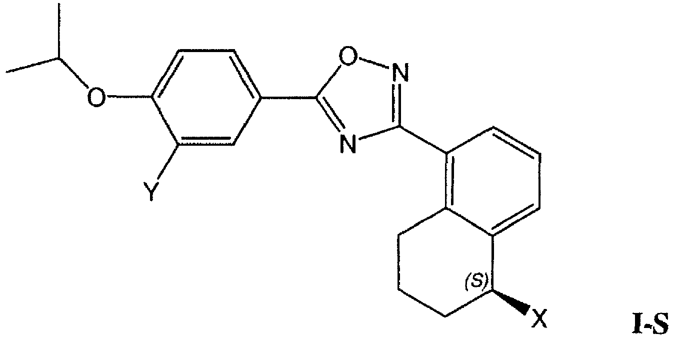

The term "derivative", a used herein, includes entities structurally derived from a given compound i.e. a chemical compound having undergone a chemical derivatization such as substitution or addition of a further chemical group to change (for pharmaceutical use) any of its physico-chemical properties, such as solubility or bioavailability. Derivatives include so-called prodrugs.

The term "metabolite", as used herein, refers to any compound resulting from enzymatic reactions, i.e., a compound synthesized by a process in which an enzyme takes part. In a particular embodiment, the metabolite of fingolimod is fingolimod phosphate.

The term "pharmaceutically acceptable salt" refers to any salt, which, upon administration to the recipient is capable of providing (directly or indirectly) a compound as described herein. Preferably, as used herein, the term "pharmaceutically acceptable salt" means approved by a regulatory agency of the Federal or a state government or listed in the U.S. Pharmacopeia or other generally recognized pharmacopeia for use in animals, and more particularly in humans. The preparation of salts can be carried out by methods known in the art.

For instance, pharmaceutically acceptable salts of fingolimod may be acid addition salts, base addition salts or metallic salts, and they can be synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. In a particular embodiment, the pharmaceutically acceptable salt is an acid addition salt. Examples of suitable salts are hydrochlorides, carbonates, hydrogen carbonates, acetates, lactates, butyrates, propionates, sulphates, methane sulphonates, citrates, tartrates, nitrates, sulphonates, oxalates and/or succinates.

In a more particular embodiment, the pharmaceutically acceptable salt of fingolimod is the hydrochloride salt.

In a particular embodiment, the inhibitor of sphingosine-1-phosphate receptor 1, more particularly fingolimod, an analogue, metabolite or derivative thereof, or a pharmaceutically acceptable salt thereof, is not administered together with a neurotransmitter receptor modulating agent. In a more particular embodiment, the neurotransmitter receptor modulating agent is selected from benztropine, carbetapentane, clemastine, pindolol, ipratropium, atropine, GBR12935, Snc-80, BD-1 047, salmeterol, albuterol, trifluoperazine, or a salt thereof. In an even more particular embodiment, the neurotransmitter receptor modulating agent is selected from benztropine, clemastine, salmeterol, salbutamol, trifluoperazine, or a salt thereof. In a still more particular embodiment, the neurotransmitter receptor modulating agent is selected from benztropine or a salt thereof (e.g., benztropine mesylate).

In a particular embodiment, the inhibitor of sphingosine-1-phosphate receptor 1, more particularly fingolimod, an analogue, metabolite or derivative thereof, or a pharmaceutically acceptable salt thereof, is not administered together with methylglyoxal bis(guanylhydrazone) (MGBG or mitoguazone). The term "methylglyoxal bis(guanylhydrazone)" or "MGBG" or "mitoguazone", as used herein, refers to a competitive polyamine inhibitor of S-adenosyl methionine decarboxylase (SAMDC, AMD-I), which catalyzes the synthesis of spermidine, a polyamine.

In a particular embodiment, the adrenoleukodystrophy is selected from the group consisting of adult adrenomyeloneuropathy (AMN), cerebral adrenomyeloneuropathy (cAMN) and the childhood variant of adrenoleukodystrophy (cALD).

The ALD can occur with or without inflammatory demyelination. In a particular embodiment, the ALD occurs without inflammatory demyelination.

The term "treatment and/or prevention", as used herein, means administration of the inhibitor of sphingosine-1-phosphate receptor 1, more particularly fingolimod, an analogue, metabolite or derivative thereof or a pharmaceutically acceptable salt thereof, or a pharmaceutical formulation comprising the inhibitor of sphingosine-1-phosphate receptor 1, more particularly fingolimod, an analogue, metabolite or derivative thereof or a pharmaceutically acceptable salt thereof, to preserve health in a patient suffering or in risk of suffering an adrenoleukodystrophy, preferably adult adrenomyeloneuropathy (AMN), cerebral adrenomyeloneuropathy (cAMN) or the childhood variant of adrenoleukodystrophy (cALD) adrenoleukodystrophy, even more preferably adrenomyeloneuropathy (AMN). Said terms also include administration of the inhibitor of sphingosine-1-phosphate receptor 1, more particularly fingolimod, an analogue, metabolite or derivative thereof or a pharmaceutically acceptable salt thereof, or pharmaceutical formulation comprising the inhibitor of sphingosine-1-phosphate receptor 1, more particularly fingolimod, an analogue, metabolite or derivative thereof or a pharmaceutically acceptable salt thereof, to prevent, ameliorate or eliminate one or more symptoms associated with an adrenoleukodystrophy, preferably adult adrenomyeloneuropathy (AMN), cerebral adrenomyeloneuropathy (cAMN) or the childhood variant of adrenoleukodystrophy (cALD)adrenoleukodystrophy, even more preferably adrenomyeloneuropathy (AMN).

The term "effective" amount or a "therapeutically effective amount" of a drug or pharmacologically active agent is meant a nontoxic but sufficient amount of the drug or agent to provide the desired effect. In the combination therapy for use according to the present invention defined in the appended claims, an "effective amount" of one component of the combination is the amount of that compound that is effective to provide the desired effect when used in combination with the other components of the combination. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation.

Generally the effective administered amount of the inhibitor of sphingosine-1-phosphate receptor 1, more particularly fingolimod, an analogue, metabolite or derivative thereof or a pharmaceutically acceptable salt thereof, will depend on the severity of the disorder, or the age, weight or mode of administration. In practice, the physician will determine the actual dosage and administration regimen, which will be the most suitable for the patient suffering or in risk of suffering from an adrenoleukodystrophy, preferably adult adrenomyeloneuropathy (AMN), cerebral adrenomyeloneuropathy (cAMN) or the childhood variant of adrenoleukodystrophy (cALD) adrenoleukodystrophy, even more preferably adrenomyeloneuropathy (AMN).

In another aspect, the invention relates to an inhibitor of sphingosine-1-phosphate receptor 1, more particularly fingolimod, an analogue, metabolite or derivative thereof or a pharmaceutically acceptable salt thereof for use in the treatment and/or prevention of an adrenoleukodystrophy, wherein said inhibitor of sphingosine-1-phosphate receptor 1, more particularly fingolimod, analogue, metabolite or derivative thereof, or said pharmaceutically acceptable salt thereof, is administered to a patient which has been diagnosed using the diagnostic method of the invention. In a particular embodiment, the patient is diagnosed, prior to the administration of the inhibitor of sphingosine-1-phosphate receptor 1, more particularly fingolimod, analogue, metabolite or derivative thereof, or said pharmaceutically acceptable salt thereof, using the diagnostic method of the invention.

### Pharmaceutical compositions

In a fifth aspect, the invention relates to a pharmaceutical composition, hereinafter pharmaceutical composition of the invention, comprising an inhibitor of sphingosine-1-phosphate receptor 1, and one or more drugs selected from the group consisting of an antioxidant selected from alpha-lipoic acid, vitamin E, and N-acetylcysteine, an antioxidant targeted to mitochondria, a histone deacetylase inhibitor, an inhibitor of mitochondria transition pore opening, an anti-inflammatory drug, a PPAR agonist, a RXR agonist, a sirtuin 1 agonist, an hypolipidemic drug, a fatty acid composition able to decrease circulating levels of hexacosanoic acid (C26:0) and an autophagy activator.

The terms "inhibitor of sphingosine-1-phosphate receptor 1, more particularly", "antioxidant selected from alpha-lipoic acid, vitamin E, and N-acetylcysteine", "antioxidant targeted to mitochondria", "histone deacetylase inhibitors", "anti-inflammatory drugs", "PPAR agonists", "RXR agonists "sirtuin 1 agonists", "hypolipidemic agents" and "fatty acid composition able to decrease circulating levels of hexacosanoic acid (C26:0)" have been previously defined.

In a particular embodiment, the inhibitor of sphingosine-1-phosphate receptor 1 is fingolimod, an analogue, metabolite or derivative thereof, or a pharmaceutically acceptable salt thereof.In a particular embodiment, the fingolimod analogue is selected from the group consisting of siponimod, KRP-203, ponesimod and RPC-1063. In a more particular embodiment, the fingolimod analogue is siponimod.

The term "combination" as used herein, is meant to encompass the administration to a patient suffering in a patient suffering or in risk of suffering from an adrenoleukodystrophy, preferably adult adrenomyeloneuropathy (AMN), cerebral adrenomyeloneuropathy (cAMN) or the childhood variant of adrenoleukodystrophy (cALD)adrenoleukodystrophy, even more preferably adrenomyeloneuropathy (AMN), of fingolimod, an analogue, metabolite or derivative thereof or a pharmaceutically acceptable salt thereof, and the other referred therapeutic agent previously defined, in the same or separate pharmaceutical formulations, and at the same time or at different times.

The combination drugs can be administered together, one after the other or separately in one combined unit dosage form or in two separate unit dosage forms. The unit dosage form may also be a fixed combination.

Simultaneous use (administration) may, e.g., take place in the form of one fixed combination with two or more active ingredients, or by simultaneously administering two or more active ingredients that are formulated independently.

Sequential use (administration) preferably means administration of one (or more) components of a combination at one time point, other components at a different time point, that is, in a chronically staggered manner, preferably such that the combination shows more efficiency than the single compounds administered independently.

Separate use (administration) preferably means administration of the components of the combination independently of each other at different time points.

In a preferred embodiment of the invention the inhibitor of sphingosine-1-phosphate receptor 1, more particularly fingolimod, an analogue, metabolite or derivative thereof or a pharmaceutically acceptable salt thereof, and the other drug form part of the same composition.

In another preferred embodiment, the inhibitor of sphingosine-1-phosphate receptor 1, more particularly fingolimod, an analogue, metabolite or derivative thereof or a pharmaceutically acceptable salt thereof, and the other drug are provided as separate compositions for administration at the same time or at different times.

In a particular embodiment, the pharmaceutical composition also comprises a pharmaceutically acceptable excipient.

The term "pharmaceutically acceptable excipient" refers to a diluent, adjuvant, carrier, or vehicle with which the active ingredient is administered. Such pharmaceutical excipients can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions, also buffers, isotonic agents or agents capable increasing solubility. Suitable pharmaceutical carriers are described in "Remington's Pharmaceutical Sciences" by E.W. Martin or "Tratado de Farmacia Galénica", C. Faulí i Trillo, Luzán 5, S.A. de Ediciones, 1993.

The pharmaceutical composition of the invention may be administered in the form of different preparations. Examples of pharmaceutical compositions include any solid (tablets, pills, capsules, granules etc.) or liquid (solutions, suspensions, syrups or emulsions) composition for oral, topical or parenteral administration.

In a preferred embodiment the pharmaceutical compositions are in oral form. Oral forms of pharmaceutical compositions may be solid or liquid. Suitable dosage forms for oral administration may be tablets, capsules, pills, granules, syrups or solutions. Preferably, the pharmaceutical composition is a solid form selected from the group consisting of tablets, capsules, pills, and granules; even more preferably, a tablet.

The solid oral pharmaceutical compositions may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate, hydroxypropylcellulose, carboxymethylcelluloses or microcrystalline cellulose; or pharmaceutically acceptable wetting agents such as sodium lauryl sulfate. Preferably, the excipients are selected from the group consisting of lactose monohydrate, hydroxypropylcellulose, carboxymethylcellulose calcium, and magnesium stearate.

The solid oral compositions may be prepared by conventional methods of blending, filling or tableting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

In another aspect, the invention relates to the pharmaceutical composition of the invention for use in the treatment and/or prevention of an adrenoleukodystrophy. Alternatively, the disclosure relates to the use of the pharmaceutical composition of the invention in the manufacture of a medicament for the treatment and/or prevention of an adrenoleukodystrophy. Alternatively, the disclosure relates to a method of treatment and/or prevention of an adrenoleukodystrophy in a subject comprising administering to said subject a therapeutically effective amount of the pharmaceutical composition of the invention.

In a particular embodiment, the adrenoleukodystrophy is selected from the group consisting of adult adrenomyeloneuropathy (AMN), cerebral adrenomyeloneuropathy (cAMN) and the childhood variant of adrenoleukodystrophy (cALD).

The ALD can occur with or without inflammatory demyelination. In a particular embodiment, the ALD occurs without inflammatory demyelination.

In another aspect, the invention relates to the pharmaceutical composition of the invention for use in the treatment and/or prevention of an adrenoleukodystrophy, wherein said pharmaceutical composition is administered to a patient which has been diagnosed using the diagnostic method of the invention. In a particular embodiment, the patient is diagnosed, prior to the administration of the pharmaceutical composition of the invention, using the diagnostic method of the invention. The methods of the invention and the products for medical use of the invention are defined in the appended claims.

### Example 1: Sphingosine-2-phosphase kinase and Sphingosine-1-phosphate receptor 1 levels are increased in peripheral mononuclear cells from AMN patients

In figure 1A, the inventors performed Q-PCR experiments in PBMC of AMN patients, to identify an increase of SPHK2 (sphingosine kinase 2) and S1PR1 (sphingosine 1-P receptor), two enzymes controlling the synthesis of sphingosine-1-P, to find them increased in AMN patients. In figure 1B, the inventors measured the levels of sphingosine 1-P in the plasma of AMN patients using a HPLC / ESI-Q-TOF approach. In AMN patients, the levels of sphingosine 1-P (SIP) were abnormally increased, underscoring the upregulation of the proinflammatory SIP pathway. This result constitutes a strong rationale for the use of inhibitors of the S1P pathway to treat AMN, and also, X-ALD, such as fingolimod, fingolimod analogues, metabolites, derivatives and molecules alike.

Moreover, the experiment in figure 1B shows that a 3-month treatment with a combination of antioxidants (N-acetylcysteine, vitamin E and lipoic acid), is able to normalize levels of SIP, indicating a previously unreported redox dependence of this pathway in X-ALD.

### Example 2. Altered glycolipid and glycerophospholipid signalling drive inflammatory cascades in adrenomyeloneuropathy

The inventors set out to identify a molecular signature for AMN by conducting a metabolomic/lipidomic analysis on PBMC and plasma obtained from patients and controls. The results were combined with transcriptomic data from spinal cords from the *Abcd1-* mouse model at different stages of disease progression, using an integrated bioinformatic analysis. Several dysregulated key pathways that were subsequently experimentally validated by independent, complementary techniques were pinpointed.

### Metabolomic and lipidomic analysis in plasma and PBMC from AMN patients

Plasma and PBMC from AMN patients and healthy, gender and age-matched controls were collected. Lipidomic and metabolomic analysis using mass spectrometry was used to characterise their molecular profile. As there is no single universal method for metabolite extraction, two independent protocols were used to evaluate a wide range of molecules, from polar (metabolome) to apolar (lipidome) molecules. Thus, to characterize AMN-associated changes in plasma and PBMC, nontargeted metabolomic and lipidomic analyses using a LC-Q-TOF system were performed.

For plasma, using those molecular features present in at least 50% of the samples within the same group (3008 and 5579 from metabolomic and lipidomic analyses, respectively), a clustering analysis was performed. This approach demonstrated that AMN was the main factor determining both the metabolomic and lipidomic profiles of plasma. To further explore the metabolic differences between control and AMN individuals, a multivariate statistical analysis was employed, including PCA (an unsupervised technique) and PLS-DA (a supervised technique). Both techniques uncovered AMN-specific plasma metabolomic and lipidomic signatures. The statistical analysis of plasma samples revealed that 348 molecules including metabolites and lipid species were significantly different between the genotypes, and some of these molecules were identified.

**Table 9. Plasma molecules statistically significant between adrenomyeloneuropathy and control (CTL) subjects. All identities were confirmed basing on exact mass and retention time.**

| **Compound** | **Regulation (AMN vs control)** | **Corrected p-value** | **Fold change¹** |
|---|---|---|---|
| Glycocholic acid | down | 0.029 | >10 |
| Cholic acid | down | 0.045 | >10 |
| 5-β-Cholestane-3α,7α,12α-triol | up | 0.024 | >10 |
| 5β-androstane-3,17-dione | down | 4.81E-03 | >10 |
| Succinic semialdehyde | up | 0.045 | >10 |
| N-Hexanoic Acid* | down | 1.65E-05 | 1-5 |
| Eicosapentaenoic acid | down | 0.027 | >10 |
| Hexadecanedioic acid | down | 0.017 | >10 |
| Monoacylglycerol(16:0) | down | 0.032 | >10 |
| Ganglioside GA1 (d18:1/22:0) | down | 0.042 | >10 |
| Glycerophospatidylethanolamine (43:0) | up | 0.017 | >10 |
| Glycerophospatidylserine (38:4) | down | 0.028 | 1-5 |

| | | | |
|---|---|---|---|
| * These identities were based on exact mass, retention time and MS/MS spectrum. 1 Fold change is calculated from log transformed raw intensities. Student's t test, p<0,05, with Benjamini-Hochberg Multiple Testing Correction was used. | | | |

Pathway analyses combining the molecules with a putative identity, revealed several targets and included lipid-driven inflammation-associated pathways such as ceramide degradation and sphingomyelin metabolism.

Metabolites involved in the bile acid biosynthesis pathway (cholic acid, glycocholic acid and 5-β-cholestane-3α,7α,12α-triol) were significantly different between AMN and control subjects, as found as well in the PBMC analyses. Diminished levels of three free fatty acids (n-hexanoic, eicosapentaenoic and hexadecanedioic acids) in the AMN group were also found (Table 9). Finally, succinic semialdehyde, an intermediate in the catabolism of γ-aminobutyrate that is implicated in neurotransmission, was up-regulated in samples from AMN patients.

Applying a similar approach to that used for analyzing plasma, we observed that the PBMC metabolomic profile discriminated better between groups than did the lipidomic profile. The statistical analyses revealed that 793 molecules were significantly different between the groups.

**Table 10. Peripheral blood mononuclear cells molecules statistically significant between adrenomyeloneuropathy and control (CTL) subjects.**

| **Compound** | **Regulation (AMN vs control)** | **Corrected p-value** | **Fold change¹** |
|---|---|---|---|
| 1-methylhistidine | up | 6.15E-04 | >10 |
| 1-monopalmitin | up | 0.042 | 1-5 |
| 3-amino-1-tyrosine | up | 3.59E-09 | 1-5 |
| 3-mercaptopyruvate | up | 0.044 | 1-5 |
| 5a-androstane-3,17-dione | down | 6.23E-07 | >10 |
| Cvtidinediphosphate-ethanolamine | up | 2.67E-03 | >10 |
| Dehydroascorbic acid | up | 0.043 | 1-5 |
| Elaidic acid | up | 1.33E-03 | >10 |
| Erythritol | up | 6.7E-03 | >10 |
| Arachidonic acid | up | 0.022 | >10 |
| Galactonic acid | up | 8.19E-03 | 1-5 |
| Glutathione* | up | 0.045 | 1-5 |
| Histamine | up | 0.012 | >10 |
| Hypoxanthine * | up | 4.92E-03 | >10 |
| Captrolactone | up | 2.19E-03 | 1-5 |
| Lactamide | up | 0.018 | >10 |
| Methyl linolenate | up | 2.08E-03 | >10 |
| Methyl oleate* | up | 7.05E-07 | >10 |
| Monoolein | up | 8.06E-03 | 1-5 |
| Myristic acid | up | 7.28E-05 | >10 |
| Pentadecylic acid | up | 1.75E-05 | >10 |
| Retinaldehyde | up | 9.29E-05 | >10 |
| Stearamide | up | 5.32E-10 | 1-5 |
| Ascorbic acid | down | 0.042 | 1-5 |
| 5β-Cholestane-3α,7α,24,26-tetrol | up | 0.019 | >10 |
| 5Z,8Z-tetradecadienoic acid | down | 0.034 | >10 |
| C24 sulfatide | up | 3.93E-03 | >10 |
| Cholesteryl ester 19:0 | up | 1.35E-04 | >10 |
| Cholesteryl ester 20:5 | down | 0.011 | >10 |
| Phosphatidylglucose (38:4) | down | 0.020 | >10 |
| Glycerophospatidylethanolamine (26:0) | up | 0.048 | >10 |
| Glycerophospatidylethanolamine (36:1) | down | 0.020 | >10 |
| Glycerophospatidylethanolamine (36:3)^{a} | up | 0.016 | >10 |
| Glycerophospatidylethanolamine (36:3)^{a} | up | 0.038 | >10 |
| Glycerophospatidylethanolamine (40:6) | up | 0.011 | >10 |
| Glycerophospatidylethanolamine (40:6) | up | 0.023 | >10 |
| Glycerophospatidylethanolamine (42:1) | up | 5.28E-03 | >10 |
| Glycerophospatidylethanolamine (43:6) | down | 0.020 | >10 |
| Glycerophospatidylethanolamine (43:6) | up | 4.33E-03 | >10 |
| Glycerophospatidylethanolamine (O-16:0/22:5) | down | 9.37E-03 | >10 |
| Lactosylceramide (d18:1/12:0) | up | 0.023 | >10 |
| Lactosylceramide (d18:1/25:0) | up | 5.95E-03 | >10 |
| Diacylglycerol(32:2) | down | 0.027 | >10 |
| Triacylglycerol(40:0) | up | 0.019 | >10 |
| Triacylglycerol(51:2) | up | 0.028 | >10 |
| Triacylglycerol(60: 1) | up | 0.014 | >10 |
| Triacylglycerol(62: 1) | up | 0.021 | 5-10 |
| Triacylglycerol(62:2) | up | 5.12E-03 | 5-10 |
| Triacylglycerol(62:3) | up | 0.016 | >10 |
| Triacylglycerol(62:4) | up | 0.042 | >10 |
| Triacylglycerol( (63:0)^{b} | down | 0.05 | >10 |
| Triacylglycerol( (63:0) ^{b} | up | 9.29E-04 | >10 |
| Triacylglycerol(63:2) | up | 5.37E-09 | >10 |
| Triacylglycerol(64:0) | up | 9.31E-03 | >10 |
| Triacylglycerol(64: 1) | up | 8.42E-04 | >10 |
| Triacylglycerol(64: 10) | up | 8.10E-05 | >10 |
| Triacylglycerol( (64:4)^{c} | up | 3.29E-03 | >10 |
| Triacylglycerol( (64:4)^{c} | up | 0.033 | >10 |
| Triacylglycerol( (64:4)^{c} | up | 0.013 | >10 |
| Triacylglycerol(64:8) | up | 1.05E-03 | >10 |
| Triacylglycerol(65:1) | up | 6E-03 | >10 |
| Triacylglycerol(65:2) | up | 0.013 | >10 |
| Triacylglycerol(65:3) | down | 0.033 | >10 |
| Triacylglycerol(66:0) | up | 6.07E-03 | >10 |
| Triacylglycerol(66:2) | up | 8.13E-15 | >10 |
| Triacylglycerol(66: 6) | up | 2.54E-04 | >10 |

| | | | |
|---|---|---|---|
| All identities were confirmed basing on exact mass and retention time. *These identities were based on exact mass, retention time and MS/MS spectrum. ^{a, b, c:} Isobaric molecules with different retention time. ¹Fold change is calculated from log transformed raw intensities. For metabolomics and lipidomics, Student's t test, p<0.05, with Benjamini-Hochberg Multiple Testing Correction was used. | | | |

Table 10 lists those molecules with a putative identification. Notably, higher histamine concentrations were present in the AMN PBMC samples than in control samples, and hypoxanthine, the product of xanthine oxidase, was also present at an increased level. Supporting the suggestion that there is disturbed bile acid metabolism in AMN, 5β-cholestane-3α,7α,12α,26-tetrol levels were significantly higher in the PBMC samples of AMN patients than in control samples. Intriguingly, glycolipids such as lactosylceramide (LacCer), and most of the glycerophosphatidylethanolamine pathway metabolites (phosphatidylserine, phosphatidylethanolamine and CDP-ethanolamine), and triglyceride species were increased in the AMN patients (Table 10). Pathway analyses of the molecules with a putative identity using the Consensus-Path platform, revealed several nodes which included proinflammatory cascades driven by bioactive lipids pathways such as ceramide degradation, sphingomyelin metabolism and eicosanoid biosynthesis including metabolites from the leukotriene, prostaglandin and thromboxane subfamilies. Collectively, the vast majority of all identified metabolites can be associated with inflammation and/or redox homeostasis.

### Integrated analysis of '-omics' data in X-ALD

The metabolomic and lipidomic results were next integrated with transcriptome data from the spinal cords of *Abed1-* mice, with the aim of uncovering core molecular footprints of the disease across different species and cell types, which could be of relevance for disease pathogenesis. The common dysregulated pathways between the transcriptomes of X-ALD mouse spinal cords at 3.5, 12 and 22 months and the metabolomes of AMN patients, in PBMC and plasma were analyzed. Three shared dysregulated pathways stand out: i) the metabolism of lipids and lipoproteins, ii) signaling by GPCR (G-protein coupled receptors) and iii) sphingolipid metabolism. Pathways i) and ii) are also dysregulated in plasma. For precise visualization of the metabolic reactions, a metabolic map from Kegg (http://www.genome.jp/kegg-bin/show_pathway?map01100v) was used to build the inventor's own AMN metabolome map. This integrated analysis approach yielded several nodes of disturbance in which both the enzyme's expression (from *Abcd1-* mouse spinal cords) and their reaction products or substrates (from AMN patient's plasma or PBMC) were trending in the same direction. Four areas of concerted dysregulation of enzyme metabolite pairs were identified. The first is the synthesis of lactosylceramide (LacCer) from glucosylceramide via beta-1,4-galactosyltransferase (B4GALT6). Expression of this enzyme was raised in the transcriptomic analysis, and also in Q-PCR validatory assays in spinal cords (Fig. 2A). The second node highlights a dysregulation of glycerophospholipid metabolism, with CDP-ethanolamine as a substrate, phosphatidylethanolamine as a product and the ethanolamine phosphotransferase CEPT1 as the catalytic enzyme. Furthermore, phosphatidylethanolamine is converted to phosphatidylserine by phosphatidylserine synthase. CEPT1 also catalyzes the final step in the synthesis of phosphatidylcholine by transferring phosphocholine from CDPcholine to diacylglycerol. The resultant phosphatidylcholine is metabolized to arachidonic acid by the calcium-independent, cytosolic phospholipase 2γ, cPLA₂γ (PLA2G4C). This is an enzyme family which hydrolyses glycerophospholipids to produce free fatty acids and lysophospholipids, both of which serve as precursors in the production of signaling molecules and second messengers of inflammatory processes such as eicosanoids and diacylglycerol. Both the *Pla2g4c* and *Cept1* transcripts were raised in the transcriptomics analysis of spinal cords, and were confirmed in the validatory Q-PCR analysis of *Abcd1-* spinal cords that ensued (Fig. 2A), already very early in life, at 3 months of age (Fig. 2A). The third area of concerted dysregulation is the formation of retinal (retinaldehyde) by retinol dehydrogenase 11 (RDH11), on the □□□hway of retinoic acid biosynthesis from retinol (vitamin A). This increase in is correlated to increased expression of the *Rdh11* transcript levels in the transcriptome of *Abcd1-* spinal cords; and in the validatory Q-PCR analysis of AMN patients's mononuclear cells (Fig. 2B) and in the spinal cords of *Abcd1-* mice (Fig. 2A). RDH11 is a membrane bound enzyme induced by sterol-regulatory element binding proteins (SREBPs), and proposed to reduce in addition to retinal, other toxic fatty aldehydes such as the oxidation product 4-hydroxynonenal (4-HNE), which has been found in human X-ALD samples. RDH11 malfunction causes a human syndrome involving brain and retina development, which highlights the importance of the enzyme. The fourth node puts the accent on the biosynthesis of bile acids, with lower levels of cholic and glycocholic acids, which are final products of peroxisomal β-oxidation. This is concordant with dysregulated expression of the peroxisomal bifunctional protein and the racemase AMACR enzymes in the *Abcd1-* mouse spinal cords. Upstream in the same pathway, we find an accumulation of the 5-β-cholestane-3α,7α,12α-triol and 5β-cholestane-3α,7α,12α,26-tetrol, cholesterol intermediates in the bile synthesis pathway. The enzyme which catalyzes the oxidation step from the of 5-β-cholestane-3α,7α,12α-triol to 5β-cholestane-3α,7α,12α,26-tetrol is the sterol 27 hydroxylase (CYP27A1), a mitochondrial member of the cytochrome P450 superfamily. CYP27A1 transcript levels were increased in PBMC (Fig. 6B), as predicted in the transcriptome analysis of spinal cords. Notably, the inactivation of this enzyme is responsible for cerebrotendinous xanthomatosis (OMIM 213700), a rare autosomal recessive lipid storage disease associated with central demyelination, ataxia and spastic paraplegia. In addition, several enzymes of fatty acid metabolism (synthesis and oxidation) were observed to be dysregulated in the transcriptome analysis, including acetyl-CoA acyltransferase 2 (mitochondrial 3-oxoacyl-Coenzyme A thiolase) (Acaa2); acetyl-CoA carboxylase alpha (Acaca); acyl-CoA dehydrogenase; short/branched chain (Acadsb); hydroxyacyl-CoA dehydrogenase (Hadh); and the hydroxyacyl-CoA dehydrogenase/3-ketoacyl-CoA thiolase/enoyl-CoA hydratase (trifunctional protein), alpha subunit (Hadha). It is believed that the concomitant alteration in the levels of several fatty acids, such as hexanoic and myristic acids, as identified by metabolomics, is also of relevance although they are not displayed in the original Kegg map.

### Inflammation and oxidative stress in blood from AMN patients

To validate the hypothesis derived from the integrative analysis of -omics data, pointing to dysregulated glycosphingolipid and glycerophospholipid metabolism, which govern inflammatory cascades, several complementary approaches were used. These were: i) a quantitative mass spectrometry panel to assess lipid mediators of inflammation and lipid peroxidation markers, mostly derivatives of arachidonic acid, in plasma (Biocrates); ii) a Q-PCR array to measure the expression of inflammatory cytokines and their cognate receptors in PBMC; and iii) an immunoassay using MILLIPLEX™ technology to identify adipokines and cytokines in plasma. The Biocrates MS/MS analyses pinpointed three inflammatory products of arachidonic acid metabolites increased in AMN samples: the eicosanoids thromboxane B2, and 12- and 15- hydroxyeicosatetraenoic acids (TXB2, 12S-HETE and 15S-HETE) (Fig. 2C). As the products of arachidonic acid metabolism such as leukotriene B4, 1-palmitoyl-2-oleoylsn- glycero-3-phosphocholine, 4-hydroxy-2-nonenal (4-HNE), 15S-HETE and 9/13- HODEs are potent ligands of peroxisome proliferator-activated receptor PPARα, PPARβ/δ and PPARγ, the expression of these master regulators of lipid metabolism and inflammation were analyzed by Q-PCR. The results show that *PPAR*β/δ but not *PPAR*α or *PPAR*γ were up-regulated in PBMC from AMN patients (Fig. 2B) and *PPARγ* was lowered in *Abcd1-* spinal cords at 12 month of age (Fig. 2A).

The inventors next set out to investigate correlative levels of cytokines that would underscore the inflammatory process suggested by the 'omics' integrative analyses. Using Milliplex technology raised levels of some inflammatory cytokines (HGF, IL6, IL8, MCP-1 and TNFα) and lower levels of adiponectin in plasma from AMN patients (Fig. 6D) were observed. This is suggestive of a manifest pro-inflammatory profile in AMN plasma. The latter adipokine is anti-inflammatory hormone secreted by the adipose tissue of newly uncovered relevance in neurodegeneration, which we also found decreased in the plasma of the *Abcd1-* mouse model. Next, the expression of several cytokines and their receptors in PBMC from AMN patients was examined. A cytokine array was used, showing that several molecules involved in T helper 1 (Th1) (pro-inflammatory) and/or Th2 (more protective) polarization, were increased in PBMC from AMN patients (Fig. 3A). The inflammatory profile data were complemented by using targeted Q-PCR to assess the expression of members of the suppressor of cytokine signaling (SOCS) and signal transducer and activator of transcription (STAT) families (STAT1, STAT6 and SOCS3) (Fig. 3B). In addition to controlling the expression of inflammatory cytokines, the SOCS and STAT family genes also control the polarization of Th cells toward a Th1, Th2, or Th17 phenotype, thus playing a central role in adaptive immune responses with autocrine and paracrine immunomodulatory capacities. Intriguingly, in PBMC from AMN patients, *STAT1,* which drives a proinflammatory Th1 differentiation response by immune cells, was found to be was up-regulated, whereas *SOCS3* and *STAT6,* which are involved in Th2 maturation, together with *IL4,* also had increased levels (Fig. 3B). Cytokine profile expression of PBMC showed activation of inflammation *via* the IL36 pathway *(IL36A, IL36B* and *IL36G),* instead of the classical IL1/TNFα/IL6 pathway. Furthermore, up-regulation of the *IL9*/*IL9R* as well as *IL4, IL5*/*IL5R, IL10* and *IL13,* which are Th2 markers (Fig. 3A) was also detected. In conclusion, the cytokine gene profile in AMN patients was not strongly directed towards either a Th1, Th17 or a Th2 response but was suggestive of a more generalized inflammatory imbalance.

### Example 3: Effect of fingolimod and siponimod treatment in Abcd1⁻/Abcd2^{-/-} mice Methods

### Locomotor testing

### Treadmill test

Treadmill apparatus consisted of a variable speed belt varying in terms of speed and slope. An electrified grid was located to the rear of the belt on which footshocks (0.2 mA) were administered whenever the mice fell off the belt. The treadmill apparatus (Panlab, Barcelona, Spain) consisted of a belt (50 cm long and 20 cm wide) varying in terms of speed (5 to 150 cm/s) and slope (0-25°) enclosed in a plexiglass chamber. The latency to falling off the belt (time of shocks in seconds) and the number of received shocks were measured. The mice were placed on the top of the already moving belt facing away from the electrified grid and in the direction opposite to the movement of the belt. Thus, to avoid the footshocks, the mice had to locomote forward.

The mice were evaluated in five trials in a single-day session. In the first trial, the belt speed was set at 20 cm/s and the inclination at 5°. In the second and third trial, the belt speed was 10 cm/s and the slope was increased to 10° and 20°, respectively. Then, for the fourth and the fifth trials, the inclination was maintained at 20° and the belt speed was increased to 20 and 30 cm/s, respectively. For the three first trials, mice ran 1 minute. For the fourth and fifth tests, time of the experiment was 3 and 7 minutes, respectively. The time between each test was 1, 1, 5 and 20 minutes, respectively. When the mice were subjected to consecutive trials at increasing speeds up to 20 cm/sec and a 20° slope, no differences were detected from one session to another between the WT and *Abcd1⁻*/*Abcd2*^{*-*/*-*} mice. However, when the belt speed was increased up to 30 cm/sec and the slope was 20°, differences were detected between the *Abcd1⁻*/*Abcd2*^{*-*/*-*} mice and the controls because this task requires greater coordination. These conditions were therefore chosen to assess the effects of fingolimod and siponiomod.

The training session performance was normal for all groups, indicating that correct acquisition of the skill had occurred (data not shown). The ratio between the time of shocks and the number of shocks was used as a locomotor deficit index.

### Horizontal bar cross test

The bar cross test was carried out using a wooden bar of 100 cm in length and 2 cm in width (diameter). This bar is just wide enough for mice to stand on with their hind feet hanging over the edge such that any slight lateral misstep will result in a slip. The bar was elevated 50 cm from the bench surface, so that animals did not jump off, yet were not injured upon falling from the bar. The mice were put on one end of the bar and expected to cross to the other end. To eliminate the novelty of the task as a source of slips, all animals were given four trials on the bar the day before and at the beginning of the testing session. In an experimental session, the number of hind limb lateral slips and falls from the bar was counted on four consecutive trials. If an animal fell, it was placed back on the bar at the point at which it fell and was allowed to complete the task. The bar was cleaned with ethanol after each animal.

### Statistical analysis

The data are presented as the mean ± standard deviation (SD). Significant differences were determined by Student's t-test or one-way ANOVA followed by Tukey HSD *post hoc* (**P*<0.05, ***P*<0.01, ****P*<0.001) after verifying normality. Statistical analyses were performed using the software program SPSS 12.0.

### Results

Locomotor deficits in the most used model of X-ALD, the *Abcd1⁻*/*Abcd2*^{*-*/*-*} null mice, were evaluated using treadmill and bar cross experiments after Fingolimod and Siponimod treatment, as described previously (Morató et al, Cell Death Differ. 2015 Nov;22(11):1742-53; Morató et al, Brain. 2013 Aug;136(Pt 8):2432-43) (see Figure 4). In the treadmill experiment, the double-knockout mice exhibited longer shock times and more shocks than did the WT mice. Remarkably, after four months of Fingolimod and Siponimod treatment this ratio was indistinguishable from the WT ratio (Fig. 4A). In the bar cross experiments, double-knockout mutants often failed to maintain their balance and displayed a greater tendency to slip off the bar and longer latencies to reaching the platform at the opposite end of the bar. The number of slips and the time necessary to cross the bar were also normalized following Fingolimod and Siponimod treatment (Fig. 4B.). Overall, these data show that Fingolimod and Siponimod treatment arrested the progression of the disease that occurs in the X-ALD mice.

### Example 4: Differences in the levels of the biomarkers between the severe and moderate phenotypes in AMN patients

**Table 12: Differentially expressed biomarkers between the severe and moderate phenotypes in 13 AMN patients included in a phase II clinical trial with antioxidants. Adiponectin and CXCL9 levels are lower in patients with moderate phenotype as assessed with the EDSS and 6MWT parameters, and higher in more severely affected patients. IL36A shows a different profile, being more elevated in moderate patients. EDSS is a clinical scale of spasticity; 6MWT is a clinical test measuring the distance walked in 6 minutes. Data analyzed with one-tailed paired t-test or one-tailed Wilcoxon signed rank test. Trial registered at ClinicalTrial.gov (NCT01495260).**

| | **Moderate** | **Severe** |
|---|---|---|
| **Clinical outcomes** | | |
| EDSS | 1-4.5 | 4.5-6 |
| 6MWT (1st visit) | 350-550 | 150-250 |
| ***improvement*** | 1-10% | 20-60% |

| **Cytokines** | | |
|---|---|---|
| Adiponectin | ↓ | ↑ |
| I136A | ↑ | ↓ |
| CXCL9 | ↓↓ | ↑↑ |

**Table 13: Best biomarker combinations as predictors of severity using penalized regression methods. Pearson's correlation as a means of the model prediction accuracy.**

| **Dependent variable** | **Independent variable** | | **Pearson corr.** | **P.value** | **LRT: Null** hypothesis | **LRT: p.value** |
|---|---|---|---|---|---|---|
| 6mtWT end of assay | **12S-HETE+ADIPO (before treatment)** | 6mtWT before treatment | 0.98 | 4.71E-06 | 6mtWT before treatment | 3.53E-03 |
| 6mtWT end of assay | **15S-HETE (6 months treatment)** | 6mtWT before treatment | 0.94 | 1.51E-04 | 6mtWT before treatment | 0.09286 |
| 6mtWT end of assay | **MCP-1 (ratio 6months treatment/before treatment)** | 6mtWT before treatment | 0.96 | 3.48E-05 | 6mtWT before treatment | 1.95E-02 |

A likelihood ratio test using the lmtest package was applied to differentiate the influence of the variables selected by the penalty regression methods from confounding variables such as age and the distance walked in the 6mtWT before-treatment (http://cran.r-project.org/doc/Rnews/). As a test model, generalized linear regression was performed using selected variables against the reduced (or null) nested model by using only age and 6mtWT. The power to discriminate X-ALD and controls was evaluated by calculating the area under the curve (AUC) of the receiver operating characteristic (ROC) curve. The pROC package in R was used to calculate AUCs along with their standard errors and 95% confidence intervals. All statistical analyses were performed using Bioconductor packages in R programming environment. A strong correlation is shown between the predicted 6mtWT and the real 6mtWT measured at the end of the assay, using variables measured before-treatment and after 6 months of treatment. The combination of levels of 12S-HETE and adiponectin before treatment is predictive of severity of phenotype before treatment. The levels of 15S-HETE after treatment are predictive of severity of phenotype after treatment (improvement or lack of thereof). The ratio between the levels of MCP-1 before and after treatment are predictive of response to treatment. Trial registered at ClinicalTrial.gov (NCT01495260).

All statistical analyses were performed using Bioconductor packages in R programming environment.

### Example 5: Effect of an antioxidant treatment on inflammatory markers

**Table 14: Effect of an antioxidant treatment on inflammatory markers A Significant reduction of the inflammatory markers TNF, IL8, Neopterin, 12S-HETE, 15S-HETE, TXB2, IFNA2, IL4, IL36A, CCR3 and increase of the protective cytokines Adiponectin, IL10 and CXCL9 after 6 months of antioxidant treatment, compared to before treatment. Analysed with one-tailed paired t-test or one-tailed Wilcoxon signed rank test (n=11 individual patients). Trial registered at ClinicalTrial.gov (NCT01495260).**

| | **Markers** | **Paired t-test or WSR test** |
|---|---|---|
| **Inflammatory mediators** | **Adiponectin** ↑ | **1.8E-03** |
| | **TNF↓** | **3.9E-02** |
| | **IL8** ↓ | **3.0E-02** |
| | **Neopterin** ↓ | **1.6E-02** |
| **Lipid metabolites** | **12S-HETE** ↓ | **1.9E-03** |
| | **15S-HETE** ↓ | **4.9E-03** |
| | **TXB2** ↓ | **1.4E-02** |
| **Gene expression** | **INFA2** ↓ | **9.7E-03** |
| | **IL4** ↓ | **1.9E-03** |
| | **IL10** | **4.8E-03** |
| | **IL36A** ↓ | **4.8E-03** |
| | **CCR3**↓ | **4.8E-03** |
| | **CXCL9**↑ | **3.2E-02** |

### Example 6: Accuracy of a predictive AMN model based on the levels of the biomarkers

To support the validity of the proposed molecules as potential biomarkers of AMN, receiver operating characteristic (ROC) curve analyses were performed by using the Metaboanalist platform (Xia, J., Sinelnikov, I., Han, B., and Wishart, D.S. (2015) MetaboAnalyst 3.0 - making metabolomics more meaningful . Nucl. Acids Res. 43, W251-257*).* This platform was employed using the metabolites present in cell lysates and plasma both in positive and negative ionization. The results reveal an area under the ROC curve for sphingosine 1 phosphate of 0.976 (95% confidence interval, from now on CI 0.882-1), showing its potential for this purpose. As calculated from the above measurements, an optimal cutoff value of 21900 ms counts at the specified m/z value and retention time of sphingosine-1-phosphate, adjusted by internal standards, results in a 100% sensitivity (or 100% true positive rate) with a 84% of specificity (15.4% false positive rate).

By using cross-validation analyses (100, by using a linear vector supported method) it is disclosed that most of the samples were correctly attributed, with only one control sample attributed to AMN group. Further, average accuracy based on 100 cross validations is 0.892 (near 90% accuracy), with an accuracy for held out data of 0.8.

To evaluate whether further variables were needed to improve overall quality of model, similar tests were performed employing other biomarkers present in the database. ROC curves were generated by Monte-Carlo cross validation (MCCV) using balanced subsampling. In each MCCV, two thirds (2/3) of the samples are used to evaluate the feature importance. The top 2, 3, 5, 10 ...100 (max) important variables are then used to build classification models which are validated on the 1/3 the samples that were left out. The procedure was repeated multiple times to calculate the performance and confidence interval of each model. The linear supported vector machine approach (SVM) was selected as classification method and the software built-in supported vector machine approach as a feature ranking method.

By using the other variables present in the discovery dataset (see Table 15 for predicted ROC values of the proposed biomarkers), one could design a model with a high accuracy (AUC for a model with 2 variables: 0.95 CI 0.75-1; with 3 variables: 0.976 CI 0.764-1; with 5 variables: 0.986 CI 0.827-1; with 10 variables 0.999 CI 1-1).

**Table 15. ROC area values, with 95% confidence intervals of ROC area values of the proposed metabolomic and lipidomic markers, together with optimal cutoff and sensitivity and specificity values at this cutoff, based on the data of the population analyzed.**

| **Marker** | **AUC** | **95% CI for AUC** | **Cutoff value (in ms counts)** | **Sensitivity (for given cutoff)** | **Specificity (for given cutoff)** |
|---|---|---|---|---|---|
| Sphingosine-1-phosphate | 0.976 | 0.882-1 | 21900 | 1 | 0.8 |
| 5-beta-cholestane-(3-alfa, 7-alfa, 12-alfa) triol | 0.75 | 0.583-0.875 | 2780 | 1 | 0.5 |
| Succinic semialdehyde | 0.731 | 0.509-0.917 | 3300 | 0.83 | 0.75 |
| Glycerophosphatidiylethanolamine (43:0) | 0.849 | 0.677-0.972 | 2250 | 0.9 | 0.74 |
| Glycocholic acid | 0.75 | 0.625-0.875 | 2450 | 0.5 | 1 |
| Cholic acid | 0.774 | 0.625-0.917 | 10900 | 0.6 | 1 |
| 5-beta-androstane-3,17-dione | 1 | 1-1 | 55700 | 1 | 1 |
| n-Hexanoic acid | 1 | 0.972-1 | 254000 | 0.9 | 1 |
| Eicosapentaenoic acid | 0.618 | 0.34-9.854 | 9940 | 0.6 | 1 |
| Hexadecanedioic acid | 0.903 | 0.745-1 | 15600 | 0.8 | 1 |
| Monoacylglycerol (16:0) | 0.792 | 0.667-0.917 | 3300 | 0.6 | 1 |
| Ganglioside GA1 (d18:1/22:0) | 0.948 | 0.826-1 | 8680 | 0.9 | 0.9 |
| Glycerophosphatidiylserine (38:4) | 0.854 | 0.674-0.986 | 261000 | 0.8 | 0.8 |
| 1-methylhistidine | 0.911 | 0.745-1 | 205000 | 0.8 | 0.8 |
| 1-monopalmitin | 0.611 | 0.343-0.877 | 3420 | 0.7 | 0.8 |
| 3-amino-1-tyrosine | 0.882 | 0.719-0.982 | 77600 | 0.7 | 1 |
| 3-mercaptopyruvate | 0.781 | 0.568-0.941 | 175000 | 0.7 | 0.8 |
| Cytidinediphosphate-ethanolamine | 0.811 | 0.627-0.964 | 5980 | 0.8 | 0.8 |
| Dehydroascorbic acid | 0.769 | 0.527-0.911 | 68100 | 0.7 | 0.8 |
| Elaidic acid | 0.947 | 0.827-1 | 12700 | 0.9 | 0.9 |
| Erythritol | 0.899 | 0.763-1 | 13900 | 1 | 0.8 |
| Arachidonic acid | 0.722 | 0.55-0.849 | 3660 | 0.9 | 0.5 |
| Galactonic acid | 0.852 | 0.669-0.979 | 130000 | 0.8 | 0.8 |
| Glutathione | 0.893 | 0.725-1 | 204000 | 1 | 0.8 |
| Histamine | 0.873 | 0.679-1 | 51400 | 0.9 | 0.8 |
| Hypoxanthine | 0.858 | 0.671-0.976 | 19300 | 0.7 | 0.9 |
| Caprolactone | 0.893 | 0.718-1 | 686000 | 0.9 | 0.8 |
| Lactamide | 0.719 | 0.527-0.888 | 13200 | 0.8 | 0.6 |
| Methyl linolenate | 0.864 | 0.66-0.991 | 14100 | 0.8 | 0.9 |
| Methyl oleate | 0.893 | 0.74-1 | 3250 | 0.9 | 0.9 |
| Monoolein | 0.781 | 0.544-0.926 | 1970 | 0.6 | 0.9 |
| Myristic acid | 0.982 | 0.914-1 | 14000 | 0.9 | 0.9 |
| Pentadecylic acid | 0.953 | 0.856-1 | 11000 | 1 | 0.8 |
| Retinaldehyde | 0.994 | 0.947-1 | 24000 | 1 | 0.9 |
| Stearamide | 1 | 1-1 | 235000 | 1 | 1 |
| 5beta-cholestane-(3 alfa,7 alfa), 24,26 -tetrol | 0.769 | 0.654-0.923 | 2950 | 1 | 0.5 |
| C24 sulfatide | 0.917 | 0.775-1 | 9610 | 0.8 | 0.8 |
| Cholesterylester 19:0 | 0.885 | 0.769-0.982 | 2970 | 1 | 0.8 |
| Glycerophosphatidyl ethanolamine (26:0) | 0.722 | 0.516-0.908 | 7500 | 0.8 | 0.7 |
| Glycerophosphatidyl ethanolamine (36:3) | 0.769 | 0.654-0.923 | 4040 | 1 | 0.5 |
| Glycerophosphatidiyl ethanolamine (40:6) | 0.71 | 0.495-0.901 | 5760 | 0.9 | 0.7 |
| Glycerophosphatidiyl ethanolamine (42:1) | 0.808 | 0.654-0.962 | 4610 | 1 | 0.6 |
| Glycerophosphatidiyl ethanolamine (43:6) | 0.834 | 0.669-0.959 | 4760 | 1 | 0.7 |
| Lactosylceramide (d18:1/12:0) | 0.808 | 0.609-0.938 | 4340 | 0.8 | 0.7 |
| Lactosylceramide (d18:1/25:0) | 0.808 | 0.692-0.923 | 2260 | 1 | 0.6 |
| Triacylglycerol (40:0) | 0.701 | 0.65-0.84 | 2300 | 0.5 | 1 |
| Triacylglycerol (51:2) | 0.769 | 0.615-0.885 | 2580 | 1 | 0.5 |
| Triacylglycerol (60:1) | 0.929 | 0.754-1 | 33000 | 1 | 0.9 |
| Triacylglycerol (62:1) | 1 | 1-1 | 62500 | 1 | 1 |
| Triacylglycerol (62:2) | 0.858 | 0.69-0.969 | 2260 | 0.8 | 0.8 |
| Triacylglycerol (62:3) | 0.82 | 0.65-0.89 | 2400 | 0.8 | 0.7 |
| Triacylglycerol (62:4) | 0.817 | 0.602-0.941 | 1620 | 0.8 | 0.8 |
| Triacylglycerol (63:0) | 0.769 | 0.615-0.885 | 1850 | 0.5 | 1 |
| Triacylglycerol (63:2) | 1 | 1-1 | 6090 | 1 | 1 |
| Triacylglycerol (64:0) | 0.982 | 0.92-1 | 9390 | 1 | 0.9 |
| Triacylglycerol (64:1) | 1 | 1-1 | 11700 | 1 | 1 |
| Triacylglycerol (64:4) | 0.947 | 0.843-1 | 12100 | 0.8 | 0.8 |
| Triacylglycerol (64:5) | 0.855 | 0.68-0.963 | 2830 | 0.8 | 0.8 |
| Triacylglycerol (64:8) | 0.953 | 0.834-1 | 7250 | 0.8 | 0.9 |
| Triacylglycerol (65:1) | 0.864 | 0.74-0.962 | 2830 | 0.9 | 0.8 |
| Triacylglycerol (65:2) | 0.808 | 0.692-0.923 | 2790 | 1 | 0.6 |
| Triacylglycerol (66:0) | 0.885 | 0.734-0.982 | 5860 | 1 | 0.8 |
| Triacylglycerol (66:2) | 1 | 1-1 | 6620 | 1 | 1 |
| Triacylglycerol (66:6) | 0.885 | 0.769-0.962 | 4300 | 1 | 0.8 |
| 5-alfa-androstane-3,17-dione | 1 | 1-1 | 7060 | 1 | 1 |
| Ascorbic acid | 0.828 | 0.642-0.964 | 326000 | 0.7 | 0.9 |
| 5Z,8Z-tetradecadienoic acid | 0.941 | 0.822-1 | 93800 | 0.8 | 0.9 |
| Cholesteryl ester 20:5 | 0.851 | 0.676-0.97 | 1760 | 0.8 | 0.9 |
| Glycerophosphatidylethanotamine (36:1) | 0.817 | 0.627-0.964 | 4700 | 0.7 | 0.9 |
| Glycerophosphatidylethanolamine (43:6) | 0.808 | 0.675-0.92 | 4420 | 0.5 | 1 |
| Glycerophosphatidylethanolamine (O-16:0/22:5) | 0.837 | 0.698-0.936 | 3030 | 0.6 | 1 |
| Diacylglcyerol (32:2) | 0.858 | 0.66-0.976 | 15600 | 0.8 | 0.8 |
| Triacylglycerol (63:0) | 0.769 | 0.654-0.885 | 1850 | 0.5 | 1 |
| Triacylglycerol (65:3) | 0.846 | 0.669-0.974 | 9350 | 0.8 | 0.8 |

Thus, a multivariate model with 10 potential biomarkers reaches an accuracy of 100%. Similarly, by using cross-validation analyses (100, by using a linear vector supported method) it is disclosed that 100% samples were correctly attributed.

In evaluating whether the introduction of further variables could increase the predictive accuracy, the results showed that the accuracy was increased (from 89.8% to 99.2%), from using a two variable model up to a 44 variable model.

Considering which variables were included for instance in a 2 variable system, it is shown that sphingosine 1 phosphate and TAG (63:2) were the 2 variables showing highest accuracy.

## Claims

1. A method for the diagnosis of an adrenoleukodystrophy in a subject comprising determining in a sample from said subject a value selected from the group consisting of
- the levels of sphingosine-1-phosphate,
- the expression levels of sphingosine kinase 2,
- the expression levels sphingosine-1-phosphate receptor,
wherein
increased levels of sphingosine-1-phosphate,
increased levels of sphingosine kinase 2,
increased levels of sphingosine-1-phosphate receptor,
with respect to a reference value are indicative that the subject suffers from an adreno leukodystrophy.

2. A method for monitoring the progression of an adrenoleukodystrophy in a subject suffering from adrenoleukodystrophy comprising determining in a sample from said subject a value selected from the group consisting of
- the levels of sphingosine-1-phosphate,
- the expression levels of sphingosine kinase 2,
- the expression levels sphingosine-1-phosphate receptor,
wherein
increased levels of sphingosine-1-phosphate,
increased levels of sphingosine kinase 2,
increased levels of sphingosine-1-phosphate receptor,
with respect to a value determined in a sample from the same subject at an earlier time point is indicative of a worsening of the adrenoleukodystrophy or wherein decreased levels of sphingosine-1-phosphate,
decreased levels of sphingosine kinase 2,
decreased levels of sphingosine-1-phosphate receptor,
with respect to a value determined in a sample from the same subject at an earlier time point is indicative of an amelioration of the adrenoleukodystrophy.

3. A method for monitoring the effect of an adrenoleukodystrophy therapy in a subject suffering from adrenoleukodystrophy comprising determining in a sample from said subject a value selected from the group consisting of
- the levels of sphingosine-1-phosphate,
- the expression levels of sphingosine kinase 2,
- the expression levels sphingosine-1-phosphate receptor,
wherein
increased levels of sphingosine-1-phosphate,
increased levels of sphingosine kinase 2,
increased levels of sphingosine-1-phosphate receptor,
with respect to the value determined prior to the administration of the therapy is indicative that the therapy is not effective
or wherein
decreased levels of sphingosine-1-phosphate,
decreased levels of sphingosine kinase 2,
decreased levels of sphingosine-1-phosphate receptor,
with respect to a value determined prior to the administration of the therapy is indicative that the therapy is effective.

4. The method according to claim 3 wherein the therapy comprises an antioxidant compound.

5. The method according to claim 4 wherein the antioxidant compound is a combination of N-acetylcysteine, lipoic acid and vitamin E.

6. The method according to any of claims 1 to 5 wherein the adrenoleukodystrophy occurs without inflammatory demyelination.

7. The method according to any of claims 1 to 6 wherein the sample is a sample containing peripheral mononuclear cells.

8. The method according to any of claims 1 to 7 wherein the adrenoleukodystrophy is selected from the group consisting of adult adrenomyeloneuropathy (AMN), cerebral adrenomyeloneuropathy (cAMN) and the childhood variant of drenoleukodystrophy (cALD).

9. An inhibitor of sphingosine-1-phosphate receptor 1 or a pharmaceutical composition comprising said inhibitor, both for use in the treatment and/or prevention of an adrenoleukodystrophy.

10. The inhibitor of sphingosine-1-phosphate receptor 1 or the pharmaceutical composition comprising said inhibitor, both for use in the treatment and/or prevention of an adrenoleukodystrophy according to claim 9, wherein said inhibitor is fingolimod, an analogue, metabolite or derivative thereof, or a pharmaceutically acceptable salt thereof.

11. The inhibitor of sphingosine-1-phosphate receptor 1 or the pharmaceutical composition comprising said inhibitor, both for use in the treatment and/or prevention of an adrenoleukodystrophy according to claim 10, wherein the fingolimod analogue is siponimod.

12. The inhibitor of sphingosine-1-phosphate receptor 1 or the pharmaceutical composition comprising said inhibitor, both for use in the treatment and/or prevention of an adrenoleukodystrophy according to any of claims 9 to 11, wherein the adrenoleukodystrophy is selected from the group consisting of adult adrenomyeloneuropathy (AMN), cerebral adrenomyeloneuropathy (cAMN) and the childhood variant of adrenoleukodystrophy (cALD).

13. The inhibitor of sphingosine-1-phosphate receptor 1 or the pharmaceutical composition comprising said inhibitor, both for use in the treatment and/or prevention of an adrenoleukodystrophy according to any of claims 9 to 12 wherein the inhibitor is administered in combination with one or more drugs selected from the group consisting of an antioxidant selected from alpha-lipoic acid, vitamin E and N-acetylcysteine, an antioxidant targeted to mitochondria, a histone deacetylase inhibitor, an inhibitor of mitochondria transition pore opening, an anti-inflammatory drug, a PPAR agonist, a RXR agonist, a sirtuin 1 agonist, a hypolipidemic drug, a fatty acid composition able to decrease circulating levels of hexacosanoic acid (C26:0) and an autophagy activator.

14. The inhibitor of sphingosine-1-phosphate receptor 1 or the pharmaceutical composition comprising said inhibitor, both for use in the treatment and/or prevention of an adrenoleukodystrophy according to any of claims 9 to 13 wherein said inhibitor is administered to a patient which is monitored using a method as defined in any of claims 2 to 8.

15. The inhibitor of sphingosine-1-phosphate receptor 1 or the pharmaceutical composition comprising said inhibitor, both for use in the treatment and/or prevention of an adrenoleukodystrophy according to claims 9 to 14 wherein the adrenoleukodystrophy occurs without inflammatory demyelination.

## Patentansprüche

1. Verfahren zur Diagnose einer Adrenoleukodystrophie bei einem Patienten, umfassend das Bestimmen eines Werts, der aus der Gruppe ausgewählt ist, die aus folgenden besteht, in einer Probe von dem Patienten:
- die Konzentrationen von Sphingosin-1-phosphat;
- die Expressionsniveaus von Sphingosinkinase 2;
- die Expressionsniveaus des Sphingosin-1-phosphat-Rezeptors;
wobei
erhöhte Konzentrationen von Sphingosin-1-phosphat,
erhöhte Konzentrationen von Sphingosinkinase 2,
erhöhte Konzentrationen von Sphingosin-1-phosphat-Rezeptor
in Bezug auf einen Referenzwert darauf hinweisen, dass der Patient an einer Adrenoleukodystrophie leidet.

2. Verfahren zur Überwachung des Fortschreitens einer Adrenoleukodystrophie bei einem Patienten, der an Adrenoleukodystrophie leidet, umfassend das Bestimmen eines Werts, der aus der Gruppe ausgewählt ist, die aus folgenden besteht, in einer Probe von dem Patienten:
- die Konzentrationen von Sphingosin-1-phosphat;
- die Expressionsniveaus von Sphingosinkinase 2;
- die Expressionsniveaus des Sphingosin-1-phosphat-Rezeptors;
wobei
erhöhte Konzentrationen von Sphingosin-1-phosphat,
erhöhte Konzentrationen von Sphingosinkinase 2,
erhöhte Konzentrationen von Sphingosin-1-phosphat-Rezeptor
in Bezug auf einen Wert, der in einer Probe von demselben Patienten zu einem früheren Zeitpunkt bestimmt wurde, auf eine Verschlechterung der Adrenoleukodystrophie hinweist oder wobei
gesunkene Konzentrationen von Sphingosin-1-phosphat, gesunkene Konzentrationen von Sphingosinkinase 2, gesunkene Konzentrationen von Sphingosin-1-phosphat-Rezeptor
in Bezug auf einen Wert, der in einer Probe von demselben Patienten zu einem früheren Zeitpunkt bestimmt wurde, auf eine Besserung der Adrenoleukodystrophie hinweist.

3. Verfahren zur Überwachung der Wirkung einer Adrenoleukodystrophie-Therapie bei einem Patienten, der an Adrenoleukodystrophie leidet, umfassend das Bestimmen eines Werts, der aus der Gruppe ausgewählt ist, die aus folgenden besteht, in einer Probe von dem Patienten:
- die Konzentrationen von Sphingosin-1-phosphat;
- die Expressionsniveaus von Sphingosinkinase 2;
- die Expressionsniveaus des Sphingosin-1-phosphat-Rezeptors;
wobei
erhöhte Konzentrationen von Sphingosin-1-phosphat,
erhöhte Konzentrationen von Sphingosinkinase 2,
erhöhte Konzentrationen von Sphingosin-1-phosphat-Rezeptor
in Bezug auf den Wert, der vor der Verabreichung der Therapie bestimmt wurde, darauf hinweist, dass die Therapie nicht wirksam ist, oder wobei
gesunkene Konzentrationen von Sphingosin-1-phosphat,
gesunkene Konzentrationen von Sphingosinkinase 2,
gesunkene Konzentrationen von Sphingosin-1-phosphat-Rezeptor
in Bezug auf einen Wert, der vor der Verabreichung der Therapie bestimmt wurde, darauf hinweist, dass die Therapie wirksam ist.

4. Verfahren gemäß Anspruch 3, wobei die Therapie eine Antioxidans-Verbindung umfasst.

5. Verfahren gemäß Anspruch 4, wobei die Antioxidans-Verbindung eine Kombination von N-Acetylcystein, Liponsäure und Vitamin E ist.

6. Verfahren gemäß einem der Ansprüche 1 bis 5, wobei die Adrenoleukodystrophie ohne entzündliche Demyelinisierung auftritt.

7. Verfahren gemäß einem der Ansprüche 1 bis 6, wobei die Probe eine Probe ist, die periphere mononukleäre Zellen enthält.

8. Verfahren gemäß einem der Ansprüche 1 bis 7, wobei die Adrenoleukodystrophie aus der Gruppe ausgewählt ist, die aus adulter Adrenomyeloneuropathie (AMN), zerebraler Adrenomyeloneuropathie (cAMN) und der kindlichen Variante der Adrenoleukodystrophie (cALD) besteht.

9. Inhibitor des Sphingosin-1-phosphat-Rezeptors 1 oder pharmazeutische Zusammensetzung, die den Inhibitor umfasst, beide zur Verwendung in der Behandlung und/oder Prävention einer Adrenoleukodystrophie.

10. Inhibitor des Sphingosin-1-phosphat-Rezeptors 1 oder pharmazeutische Zusammensetzung, die den Inhibitor umfasst, beide zur Verwendung in der Behandlung und/oder Prävention einer Adrenoleukodystrophie gemäß Anspruch 9, wobei es sich bei dem Inhibitor um Fingolimod, ein Analogon, Metabolit oder Derivat davon oder ein pharmazeutisch annehmbares Salz davon handelt.

11. Inhibitor des Sphingosin-1-phosphat-Rezeptors 1 oder pharmazeutische Zusammensetzung, die den Inhibitor umfasst, beide zur Verwendung in der Behandlung und/oder Prävention einer Adrenoleukodystrophie gemäß Anspruch 10, wobei es sich bei dem Fingolimod-Analogon um Siponimod handelt.

12. Inhibitor des Sphingosin-1-phosphat-Rezeptors 1 oder pharmazeutische Zusammensetzung, die den Inhibitor umfasst, beide zur Verwendung in der Behandlung und/oder Prävention einer Adrenoleukodystrophie gemäß einem der Ansprüche 9 bis 11, wobei die Adrenoleukodystrophie aus der Gruppe ausgewählt ist, die aus adulter Adrenomyeloneuropathie (AMN), zerebraler Adrenomyeloneuropathie (cAMN) und der kindlichen Variante der Adrenoleukodystrophie (cALD) besteht.

13. Inhibitor des Sphingosin-1-phosphat-Rezeptors 1 oder pharmazeutische Zusammensetzung, die den Inhibitor umfasst, beide zur Verwendung in der Behandlung und/oder Prävention einer Adrenoleukodystrophie gemäß einem der Ansprüche 9 bis 12, wobei der Inhibitor in Kombination mit einem oder mehreren Wirkstoffen verabreicht wird, die aus der Gruppe ausgewählt sind, die aus einem Antioxidans, das aus alpha-Liponsäure, Vitamin E und N-Acetylcystein ausgewählt ist, einem Antioxidans, das spezifisch zu Mitochondrien transportiert wird, einem Histon-Deacetylase-Inhibitor, einem Inhibitor der mitochondrialen Übergangsporenöffnung, einem entzündungshemmenden Wirkstoff, einem PPAR-Agonisten, einem RXR-Agonisten, einem Sirtuin-1-Agonisten, einem hypolipidämischen Wirkstoff, einer Fettsäurezusammensetzung, die die Konzentrationen von zirkulierender Hexacosansäure (C26:0) senken kann, und einem Autophagieaktivator besteht.

14. Inhibitor des Sphingosin-1-phosphat-Rezeptors 1 oder pharmazeutische Zusammensetzung, die den Inhibitor umfasst, beide zur Verwendung in der Behandlung und/oder Prävention einer Adrenoleukodystrophie gemäß einem der Ansprüche 9 bis 13, wobei der Inhibitor einem Patienten verabreicht wird, der mit Hilfe eines Verfahrens gemäß einem der Ansprüche 2 bis 8 überwacht wird.

15. Inhibitor des Sphingosin-1-phosphat-Rezeptors 1 oder pharmazeutische Zusammensetzung, die den Inhibitor umfasst, beide zur Verwendung in der Behandlung und/oder Prävention einer Adrenoleukodystrophie gemäß einem der Ansprüche 9 bis 14, wobei die Adrenoleukodystrophie ohne entzündliche Demyelinisierung auftritt.

## Revendications

1. Un procédé de diagnostic d'une adrénoleucodystrophie chez un sujet, comprenant le fait de déterminer dans un échantillon provenant dudit sujet une valeur choisie dans le groupe constitué par
- les niveaux de la sphingosine-1-phosphate,
- les niveaux d'expression de la sphingosine kinase 2,
- les niveaux d'expression du récepteur de la sphingosine-1-phosphate,
dans lequel
des niveaux accrus de la sphingosine-1-phosphate,
des niveaux accrus de la sphingosine kinase 2,
des niveaux accrus du récepteur de la sphingosine-1-phosphate,
par rapport à une valeur de référence sont indicatifs du fait que le sujet souffre d'un adrénoleucodystrophie.

2. Un procédé de surveillance de la progression d'une adrénoleucodystrophie chez un sujet souffrant d'adrénoleucodystrophie comprenant le fait de déterminer dans un échantillon provenant dudit sujet une valeur choisie dans le groupe constitué par
- les niveaux de la sphingosine-1-phosphate,
- les niveaux d'expression de la sphingosine kinase 2,
- les niveaux d'expression du récepteur de la sphingosine-1-phosphate,
dans lequel
des niveaux accrus de la sphingosine-1-phosphate,
des niveaux accrus de la sphingosine kinase 2,
des niveaux accrus du récepteur de la sphingosine-1-phosphate,
par rapport à une valeur déterminée dans un échantillon provenant du même sujet à un moment antérieur sont indicatifs d'une aggravation de l'adrénoleucodystrophie, ou dans lequel
des niveaux diminués de la sphingosine-1-phosphate,
des niveaux diminués de la sphingosine kinase 2,
des niveaux diminués du récepteur de la sphingosine-1-phosphate,
par rapport à une valeur déterminée dans un échantillon provenant du même sujet à un moment antérieur sont indicatifs une amélioration de l'adrénoleucodystrophie.

3. Un procédé de surveillance de l'effet d'une thérapie d'adrénoleucodystrophie chez un sujet souffrant d'adrénoleucodystrophie comprenant le fait de déterminer dans un échantillon provenant dudit sujet une valeur choisie dans le groupe comprenant
- les niveaux de la sphingosine-1-phosphate,
- les niveaux d'expression de la sphingosine kinase 2,
- les niveaux d'expression du récepteur de la sphingosine-1-phosphate,
dans lequel
des niveaux accrus de la sphingosine-1-phosphate,
des niveaux accrus de la sphingosine kinase 2,
des niveaux accrus du récepteur de la sphingosine-1-phosphate,
par rapport à la valeur déterminée avant la l'administration de la thérapie sont indicatifs du fait que la thérapie n'est pas efficace,
ou dans lequel
des niveaux diminués de la sphingosine-1-phosphate,
des niveaux diminués de la sphingosine kinase 2,
des niveaux diminués du récepteur de la sphingosine-1-phosphate,
par rapport à une valeur déterminée avant l'administration de la thérapie sont indicatifs du fait que la thérapie est efficace.

4. Le procédé selon la revendication 3, dans lequel la thérapie comprend un composé antioxydant.

5. Le procédé selon la revendication 4, dans lequel le composé antioxydant 1 est une combinaison de N-acétylcystéine, d'acide lipoïque et de vitamine E.

6. Le procédé selon l'une quelconque des revendications 1 à 5, dans lequel l'adrénoleucodystrophie se produit sans démyélinisation inflammatoire.

7. Le procédé selon l'une quelconque des revendications 1 à 6, dans lequel l'échantillon est un échantillon contenant des cellules mononucléaires périphériques.

8. Le procédé selon l'une quelconque des revendications 1 à 7, dans lequel l'adrénoleucodystrophie est sélectionnée dans le groupe constitué de l'adrénomyéloneuropathie adulte (AMN), de l'adrénomyéloneuropathie cérébrale (cAMN) et de la variante infantile de l'adrénoleucodystrophie (cALD).

9. Un inhibiteur du récepteur 1 de la sphingosine-1-phosphate ou une composition pharmaceutique comprenant ledit inhibiteur, à la fois pour une utilisation dans le traitement et / ou dans la prévention d'une adrénoleucodystrophie.

10. L'inhibiteur du récepteur 1 de la sphingosine-1-phosphate ou la composition pharmaceutique comprenant ledit inhibiteur, à la fois pour une utilisation dans le traitement et / ou dans la prévention d'une adrénoleucodystrophie selon la revendication 9, ledit inhibiteur étant le fingolimod, un analogue, un métabolite ou un dérivé de celui-ci, ou un sel pharmaceutiquement acceptable de celui-ci.

11. L'inhibiteur du récepteur 1 de la sphingosine-1-phosphate ou la composition pharmaceutique comprenant ledit inhibiteur, à la fois pour une utilisation dans le traitement et / ou la prévention d'une adrénoleucodystrophie selon la revendication 10, l'analogue du fingolimod étant le siponimod.

12. L'inhibiteur du récepteur 1 de la sphingosine-1-phosphate ou la composition pharmaceutique comprenant ledit inhibiteur, à la fois pour une utilisation dans le traitement et / ou la prévention d'une adrénoleucodystrophie selon l'une quelconque des revendications 9 à 11,
l'adrénoleucodystrophie étant sélectionnée dans le groupe composé de l'adrénomyéloneuropathie adulte (AMN), de l'adrénomyéloneuropathie cérébrale (cAMN) et de la variante infantile de l'adrénoleucodystrophie (cALD).

13. L'inhibiteur du récepteur 1 de la sphingosine-1-phosphate ou la composition pharmaceutique comprenant ledit inhibiteur, à la fois pour une utilisation dans le traitement et / ou la prévention d'une adrénoleucodystrophie selon l'une quelconque des revendications 9 à 12,
l'inhibiteur étant administré en combinaison avec un ou plusieurs médicaments sélectionnés dans le groupe constitué d'un antioxydant choisi parmi l'acide alpha-lipoïque, la vitamine E et la N-acétylcystéine, un antioxydant ciblant les mitochondries, un inhibiteur de l'histone désacétylase, un inhibiteur de l'ouverture des pores de transition des mitochondries, un médicament anti-inflammatoire, un agoniste PPAR, un agoniste RXR, un agoniste de la sirtuine 1, un médicament hypolipidémique, une composition d'acides gras apte à diminuer les niveaux circulants d'acide hexacosanoïque (C26:0) et un activateur d'autophagie.

14. L'inhibiteur du récepteur 1 de la sphingosine-1-phosphate ou la composition pharmaceutique comprenant ledit inhibiteur, à la fois pour une utilisation dans le traitement et / ou la prévention d'une adrénoleucodystrophie selon l'une quelconque des revendications 9 à 13,
ledit inhibiteur étant administré à un patient qui est surveillé en utilisant un procédé tel que défini dans l'une quelconque des revendications 2 à 8.

15. L'inhibiteur du récepteur 1 de la sphingosine-1-phosphate ou la composition pharmaceutique comprenant ledit inhibiteur, à la fois pour une utilisation dans le traitement et / ou dans la prévention d'une adrénoleucodystrophie selon les revendications 9 à 14, l'adrénoleucodystrophie se produisant sans démyélinisation inflammatoire.
